# EUROPEAN PATENT SPECIFICATION

(11) **EP 3 983 462 B1**
(45) Date of publication and mention of the grant of the patent: **08.01.2025**
(21) Application number: 20732214.0
(22) Date of filing: 12.06.2020
(51) Int. Cl.: C08F 251/00, A61Q 19/00, A61Q 19/10, A61K 8/91, C11D 3/00, C11D 3/22, C11D 3/37

(54) **AQUEOUS POLYMER DISPERSIONS SUITABLE AS OPACIFIERS IN LIQUID FORMULATIONS**
ALS TRÜBUNGSMITTEL IN FLÜSSIGEN FORMULIERUNGEN NÜTZLICHE WÄSSRIGE POLYMERDISPERSIONEN
DISPERSIONS DE POLYMÈRES AQUEUSES APPROPRIÉES UTILISÉES EN TANT QU'OPACIFIANTS DANS DES FORMULATIONS LIQUIDES

(30) Priority: 14.06.2019 EP 19180319
(43) Date of publication of application: 20.04.2022
(73) Proprietor: BASF SE, 67056 Ludwigshafen am Rhein (DE)
(72) Inventor: ETTL, Roland, 67056 Ludwigshafen (DE); WEISS, Thomas, 67056 Ludwigshafen (DE); KREIDER, Volker, 67056 Ludwigshafen (DE); DIETSCHE, Frank, 67056 Ludwigshafen (DE); WAGNER, Aaron, 67056 Ludwigshafen (DE)
(74) Representative: BASF IP Association
(86) International application number: PCT/EP2020/066250
(87) International publication number: WO 2020/249706

(56) References cited:
- WO-A1-2016/184808
- WO-A1-2017/032720
- WO-A1-2017/162921
- WO-A1-90/09406
- WO-A1-95/27004
- CA-A1- 2 800 927
- US-A- 4 301 017
- US-A- 4 835 212
- US-A- 5 055 541
- US-A- 5 925 722
- US-A- 6 090 884
- US-A1- 2003 187 135
- US-A1- 2006 154 088
- US-A1- 2010 324 178
- US-A1- 2012 180 970
- US-A1- 2015 060 724
- US-B1- 6 800 675

## Description

### FIELD OF THE INVENTION

The invention relates to the use of aqueous polymer dispersions as opacifier in liquid formulations, which are obtainable by radical aqueous emulsion polymerization in the presence of a degraded starch.

### BACKGROUND OF THE INVENTION

Pearlizing agents and opacifiers, respectively, are frequently used in liquid home care and personal care products, such as liquid cosmetic preparations and liquid detergent formulations, including liquid laundry detergent formulations, liquid dishwashing formulations and liquid cleaner formulations. The pearlizing agents and opacifiers improve the aesthetic appearance of such formulations and give them an especially high-quality appearance and, in case of cosmetics preparations and hand-dishwashing formulations a skin-caring look. In order to meet the high market demands with regard to sensory properties, pearlizing agents and opacifiers are accordingly continuously being developed and tested for their suitability in home care and personal care.

Usually opacifiers are based on synthetic water-insoluble polymer particles with high refractive index. Frequently, aqueous polymers dispersions or suspensions based on polystyrene or copolymers of sytrene and methacrylic acid are used for this purpose. Unfortunately, the biodegradibility of these styrene containing polymers is poor. Another problem associated with the production of these styrene containing polymer disperions is that during polymerisation deposits of solid materials, mostly coagulate are formed on the walls of the reaction vessel. These deposits, also termed fouling, impair the function of the plants. In this case, this can lead in particular to the reduction of throughput in in these plans caused by (in part) obstruction of pipelines, reduced heat/cooling transfer, local overheating of system parts and, hence, results in increased production stoppages due to required cleaning downtime.

WO 2016/169833 describes pearlizing agent and opacifier compositions for use in cosmetic compositions and detergents comprising at least one isosorbide diester, at least one isosorbide monoester and at least one fatty acid. However, these pearlizing agents require expensive starting materials. Apart from that, their performance is not particularly satisfactory.

WO 99/42490 and WO 2007/00420 describe aqueous polymer dispersions obtained by emulsion copolymerization of styrene/(meth)acrylate monomer mixtures in the presence of degraded starch and their use as sizing agents for paper and cardboards.

US 8,003,716, CA 2,800,927, US 2010/0324178, WO 2016/184808 describe starch-containing polymer dispersion which are obtained by free radical copolymerization of ethylenically unsaturated monomers in the presence of a redox initiator and a degraded starch. In the copolymers the proportion of styrene in the monomers to be polymerized is typically at most 60% by weight. The amount of starch is below 60 % by weight, based on the amount of polymerized monomers. The obtained polymers are suggested for use as sizes for papers and paper products and for the production of coverings.

US 2003/0187135 describes a starch-containing polymer dispersions obtainable by free-radical emulsion polymerization. Further described is inter alia a preparation of an aqueous polymer dispersion of 70% by weight of styrene and 30% by weight of butyl acrylate in the presence of 51% by weight of a degraded starch product.

WO 2017/162921 describes aqueous polymer dispersions obtainable by emulsion copolymerization of at least one optionally substituted styrene and at least one C₁-C₄-alkyl (meth)acrylate in the presence of at least one degraded starch having an average molecular weight Mn of less than 1000 g/mol. The relative amount of styrene in the monomoners is at most 75 % by weight and preferably below 55 % by weight. The dispersions are suggested as rheology modifiers in coating colors.

EP 0441197 describes graft copolymers, which are obtainable by copolymerization of a monomer mixture comprising monoethylenically unsaturated dicarboxylic acid and monoethylenically unsaturated carboxylic acid, in the presence of a monosaccharide, oligosaccharide, polysaccharide or modified polysaccharide in the weight ratio (A):(B) of (95 to 20):(5 to 80). The obtained graft copolymers are suggested for the use as anti graying agent in detergents and cleaners. Due to their monomer compositions they are water soluble.

US 7,875,359 describes self-stabilizing polymer dispersions obtainable by solution poylmerization of at least one acid-containing monomer and a hydrophobic monomer in the presence of water, the at least one acid-containing monomer being at least partially neutralized before or during polymerization. The obtained copolymers are suggested for encapsulating laundry detergents, personal care actives and dishwasher acitives, and also thereby providing opacity to liquid detergent formulations and personal care formulations.

WO 95/27004 describe starch-based opacifying agents for foods and beverage obtained by cooking a starch under conditions to solublizie the starche and adding a conventional inorganic opacifier, such as titanium dioxide, calcium citrate or calcium carbonate, under controlled conditions of temperature an shear.

US 4,301,017 describes aqueous dispersions of starch graft-copolymers. Specifically, in example III is disclosed the preparation of an aqueous polymer dispersion by polymerization of a monomer mixture of 90% styrene and 10% acrylonitrile in a thinned starch. The weight ratio of starch to monomers is 100:40, i.e. the relative amount of starch is approximately 250% with respect to the amount of monomers.

The currently known pearlizing agents and opacifiers may suffer from several disadvantages, because they show an unsatisfactory performance as pearlizing agent or opacifiers or have poor biodegradibility. Apart from that, pearlizing agents and opacifiers based styrene containing polymer disperions are difficult to produce because of the above described fouling which may occur during their production.

Accordingly, it is an object of the invention to overcome these disadvantages. In particular it is an object of the invention to provide a material, which has a high opacity effect in home care and personal care products and which is at least partly biodegradable. Moreover, such a material should be easy and inexpensive to produce.

These and further objectives are solved by aqueous polymer dispersions of ethylenically unsaturated monomers M comprising at least 70% by weight, in particular at least 75% by weight, preferably at least 80% by weight, more preferably at least 85% by weight, especially at least 89% by weight, based on the total weight of monomers M, of monovinylaromatic monomers where the aqueous polymer dispersion is obtainable by radical aqueous emulsion polymerization of said monomers M in the presence of 80 to 200%, in particular 85 to 170% by weight, especially 95 to 155% by weight, based on the total amount of monomers M, of a degraded starch.

### SUMMARY OF THE INVENTION

The invention therefore relates to the use of aqueous polymer dispersions, which are obtainable by radical aqueous emulsion polymerization of
i) ethylenically unsaturated monomers M comprising or in particular consisting of
   a) at least 70% by weight, in particular at least 75% by weight, preferably at least 80% by weight, more preferably at least 85% by weight, especially at least 89% by weight, based on the total weight of monomers M, of at least one monomer Ma, selected from the group consisting of monovinylaromatic monomers, and optionally
   b) up to 30% by weight, in particular up to 25% by weight, preferably up to 20% by weight, more preferably up to 15% by weight, especially up to 11% by weight, based on the total weight of monomers M, of one or more ethylenically unsaturated monomers Mb, which are different from the monomer Ma;
   in the presence of
ii) 80 to 200% by weight, in particular 85 to 170% by weight, especially 95 to 155% by weight, based on the total amount of monomers M, of a degraded starch as opacifiers in liquid formulations, in particular in liquid detergent formulations.

The aqueous polymer dispersion used according to the invention can be produced by a process, which comprises a radical emulsion polymerization of the aforementioned ethylenically unsaturated monomers M in water in the presence of 80 to 200% by weight, in particular 85 to 170% by weight, especially 95 to 155% by weight, based on the total amount of monomers M, of a degraded starch, in particular an enzymatically degraded starch and especially a maltodextrin.

The invention relates in particular to the use of an aqueous polymer dispersion, which is obtainable by radical aqueous emulsion polymerization of ethylenically unsaturated monomers M as defined herein in the presence of 80 to 200% by weight, in particular 85 to 170% by weight, especially 95 to 155% by weight, based on the total amount of the monomers M, of a degraded starch as an opacifier in liquid formulations, in particular in liquid detergent formulations.

The invention relates in particular to the use of the aqueous polymer dispersion as defined herein, where the monomer Ma is styrene or comprises at least 95% by weight of styrene, based on the total amount of monomers Ma.

The invention relates in particular to the use of the aqueous polymer dispersion as defined herein, where the monomers Mb comprise at least one monoethylenically unsaturated acidic monomer Mb1, selected from the group consisting of monoethylenically unsaturated monocarboxylic acids having 3 to 6 carbon atoms and monoethylenically unsaturated dicarboxylic acids having 4 to 8 carbon atoms.

The invention relates in particular to the use of the aqueous polymer dispersion as defined herein, where the monomer Mb is methacrylic acid or comprises at least 95% by weight of methacrylic acid, based on the total amount of monomers Mb.

The invention relates in particular to the use of the aqueous polymer dispersion as defined herein, where the monomers M comprise
a) 70 to 99.9% by weight, in particular 75 to 99% by weight, especially 80 to 98% by weight or 80 to 95% by weight or 85 to 95% by weight, and most preferably 89 to 93% by weight, based on the total weight of the monomers M, of at least one monomer Ma, and
b.1) 0.1 to 30% by weight, in particular 1 to 25% by weight, especially 2 to 20% by weight or 5 to 20% by weight or 5 to 15% by weight, and most preferably 7 to 11% by weight, based on the total weight of the monomers M, of at least one monomer Mb1, selected from the group consisting of monoethylenically unsaturated monocarboxylic acids having 3 to 6 carbon atoms and monoethylenically unsaturated dicarboxylic acids having 4 to 6 carbon atoms and
b.2) optionally up to 29.9% by weight, in particular 0 to 24% by weight and especially 0 to 18% by weight or 0 to 15% by weight or 0 to 10% by weight or 0 to 5% by weight or 0 to 2% by weight, based on the total weight of monomers M, of one or more further monomers ethylenically unsaturated monomers Mb2, which are different from the monomer Ma and Mb1.

The invention relates in particular to the use of the aqueous polymer dispersion as defined herein, where the aqueous polymer dispersion is obtainable by radical aqueous emulsion polymerization of monomers M comprising
a) 80 to 98% by weight or 80 to 95% by weight or 85 to 95% by weight, and most preferably 89 to 93% by weight, based on the total weight of the monomers M, of at least one monomer Ma, which comprises at least 95% of styrene, and
b.1) 2 to 20% by weight or 5 to 20% by weight or 5 to 15% by weight, and most preferably 7 to 11% by weight, based on the total weight of the monomers M, of at least one monomer Mb1, which is preferably selected from the group consisting of monoeth-ylenically unsaturated monocarboxylic acids having 3 to 6 carbon atoms and monoethylenically unsaturated dicarboxylic acids having 4 to 8 carbon atoms and which is particularly selected from the group consisting of methacrylic acid and mixtures thereof with acrylic acid. In particular at least 80% by weight, in particular at least 95% by weight, based on the total amount of monomers Mb1 is methacrylic acid;
b.2) optionally 0 to 5% by weight or 0 to 2% by weight, based on the total weight of the monomers M, based on the total weight of monomers M, of one or more further monomers ethylenically unsaturated monomers Mb2, which are different from the monomer Ma and Mb1,
in the presence of 85 to 170% by weight, especially 95 to 155% by weight, based on the total amount of the monomers M, of a degraded starch, in particular in the presence of a maltodextrin.

The invention relates in particular to the use of the aqueous polymer dispersion as defined herein, where the dispersed polymer particles have a volume median particle diameter D (v, 0.5) as determined by static light scattering of at least 150 nm, in particular at least 170 nm, more preferably at least 180 nm and especially at least 190 nm or at least 200 nm, and where the D(v, 0.5) value is in in particular in the range from 150 to 1000 nm, more preferably in the range from 170 to 900 nm or in the range from 180 to 800 nm and especially in the range from 190 to 750 nm or in the range from 200 to 700 nm.

The invention relates in particular to the use of the aqueous polymer dispersion as defined herein, where the dispersed polymer particles have a D[4,3] value of at least 220 nm, preferably at least 250 nm, in particular at least 300 nm, especially at least 350 nm, e.g. in the range from 220 to 1200 nm, preferably in the range from 250 to 1000 nm, in particular in the range from 300 to 900 nm and especially in the range from 350 to 800 nm.

The invention relates in particular to the use of the aqueous polymer dispersion as defined herein, where the particle size distribution of the dispersed particles in the aqueous polymer dispersion is polymodal.

The invention relates in particular to the use of the aqueous polymer dispersion as defined herein, where at least 15% by volume, in particular from 15 to 60% by volume of the polymer particles have a particle size of at least 300 nm, in particular of at least 350 nm, especially of at least 400 nm, e.g. in the range from 300 to 2500 nm, in particular in the range from 350 to 2000 nm, especially in the range from 400 to 1900 nm.

The invention relates in particular to the use of the aqueous polymer dispersion as defined herein, where the degraded starch has a weight average molecular weight in the range from 800 to 30000 Dalton, in particular in the range from 1000 to 10000 Dalton as determined by gel permeation chromatography.

The invention relates in particular to the use of the aqueous polymer dispersion as defined herein, where the degraded starch has a number average molecular weight in the range from 350 to 1000 g/mol as determined by gel permeation chromatography.

The invention relates in particular to the use of the aqueous polymer dispersion as defined herein, where the degraded starch has a number average molecular weight in the range from 300 to 2000 Dalton, in particular in the range from 350 to 1500 Dalton, as determined by osmometry.

The invention relates in particular to the use of the aqueous polymer dispersion as defined herein, where the degraded starch has a dextrose equivalent according to Lane and Eynon in the range from 2 to 40%, in particular in the range from 20 to 40%.

The invention relates in particular to the use of the aqueous polymer dispersion as defined herein, where the degraded starch has a dextrose equivalent according to Luff-Schoorl in the range from 2.8 to 7.5 and especially in the range from 3.0 to 7.0

The invention relates in particular to the use of the aqueous polymer dispersion as defined herein, where the degraded starch is an enzymatically degraded starch, in particular a maltodextrin.

The invention relates in particular to the use of the aqueous polymer dispersion as defined herein, where the liquid formulation is a liquid detergent formulation or a liquid cosmetic preparation.

The invention relates in particular to the use of the aqueous polymer dispersion as defined herein, where the liquid formulation comprises the polymer dispersion in an amount from 0.1 to 10% by weight, in particular from 0.5 to 5% by weight, especially 1 to 3% by weight, based on the total weight of the liquid formulation and calculated as polymer solids.

### DETAILED DESCRIPTION OF THE INVENTION

The features and ranges indicated here as "preferred", "more preferred", "particular", "special" etc. apply to the application, process and polymer dispersions in the same way, unless otherwise stated.

The terms "biodegradation" or "biodegradability" are synonyms and mean in the sense of the invention that the polymers decompose in an appropriate and demonstrable period of time when exposed to the effects of the environment. The degradation mechanism can be hydrolytic and/or oxidative, and is based mainly on exposure to microorganisms, such as bacteria, yeasts, fungi, and algae. A substance will be considered "biodegradable" if the substance in question, the polymer dispersion or the polymers contained therein here, in the test of OECD Guideline 301B from 1992 (measurement of evolution of CO₂ on composting in a mineral slurry and comparison with the theoretical maximum possible evolution of CO₂) after 28 days and 25°C undergoes biodegradation of at least 5%, particularly at least 10% and especially at least 20%.

For the purposes of the present invention, the expression "alkyl" encompasses linear and branched alkyl groups, especially having 1 to 30 carbon atoms, i.e., for "C₁-C₃₀ alkyl"; preferably the expression "alkyl" encompasses linear and branched alkyl groups having 1 to 20 carbon atoms, i.e., for "C₁-C₂₀ alkyl".

Suitable short-chain alkyl groups are, for example, linear or branched C₁-C₇ alkyl, preferably C₁-C₆ alkyl, and more preferably C₁-C₄ alkyl groups. These include, in particular, methyl, ethyl, propyl, isopropyl, n-butyl, 2-butyl, sec-butyl, tert-butyl, n-pentyl, 2-pentyl, 2-methylbutyl, 3-methylbutyl, 1,2-dimethylpropyl, 1,1-dimethylpropyl, 2,2-dimethylpropyl, 1-ethylpropyl, n-hexyl, 2-hexyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-2-methylpropyl, n-heptyl, 2-heptyl, 3-heptyl, 2-ethylpentyl, 1-propylbutyl, etc.

Suitable longer-chain alkyl groups are, for example, linear and branched C₈-C₃₀ alkyl groups, preferably C₈-C₂₀ alkyl groups. Preferably, these are predominantly linear alkyl radicals, of the kind also occurring in natural or synthetic fatty acids and fatty alcohols, and also oxo-process alcohols. They include, for example, n-octyl, n-nonyl, n-decyl, n-undecyl, n-dodecyl, n-tridecyl, n-tetradecyl, n-pentadecyl, n-hexadecyl, n-heptadecyl, n-octadecyl, and n-nonadecyl. The expression "alkyl" encompasses unsubstituted and substituted alkyl radicals.

The statements above relating to alkyl are valid *mutatis mutandis* also for the alkyl groups in alkanol, alkylamine, alkanecarboxylic acids, and alkyl esters.

The expression "allyl" for the purposes of the present invention refers to a -(CH₂)-CH=CH₂ group.

The term "monoethylenically unsaturated C₃-C₈ monocarboxylic acid" refers to a monobasic carboxylic acid having 3 to 8 C atoms, which has one ethylenically unsaturated C=C double bond. Preferred monoethylenically unsaturated C₃-C₈ monocarboxylic acids are selected from acrylic acid, methacrylic acid, vinylacetic acid, crotonic acid and mixtures thereof.

The term "monoethylenically unsaturated C₄-C₈ dicarboxylic acid" refers to a dibasic carboxylic acid having 4 to 8 C atoms, which has one ethylenically unsaturated C=C double bond. Preferred monoethylenically unsaturated C₄-C₈ dicarboxylic acids are selected from maleic acid, fumaric acid, itaconic acid, or citraconic acid and mixtures thereof.

The aqueous polymer dispersions according to the invention is obtained by radical emulsion polymerisation of ethylenically usaturated monomers M.

The monomers M comprises at least one monomer Ma as defined herein. In accordance with the invention the amount of the monomers Ma, based on the total weight of monomers M, is at least 70% by weight, in particular at least 75% by weight, especially at least 80% by weight or at least 85% by weight and may be up to 100% by weight.

The monomers Ma may be the sole or essentially sole monomer M, i.e. the amount of monomers Ma may be 100% by weight or at least 99.9% by weight, based on the total amount of the monomers M. In this group of embodiments, the amount of monomers Mb is consequently at most 0.1% by weight and may be as low as 0% by weight, based on the total amount of monomers M.

In a preferred group of embodiments the monomers M comprise at least one ethylenically unsaturated monomer which is different from the monomers Ma. The total amount of monomers Mb will however not exceed 30% by weight, in particular 25% by weight and especially 20% by weight or 15% by weight, based on the total amount of monomers M. In this preferred group of embodiments, the amount of monomers Ma is usually in the range of from 70 to 99.9% by weight, particularly 75 to 99% by weight, especially 80 to 98% by weight or 80 to 95% or 85 to 95% by weight, and most preferably 89 to 93% by weight, based on the total amount of the monomers M. Consequently, the amount of monomers Mb is then in the range from 0.1 to 30% by weight, in particular in the range from 1 to 25% by weight and especially in the range from 2 to 20% by weight or in the range from 5 to 20% by weight or in the range from 5 to 15% by weight, and most preferably 7 to 11% by weight, based on the total weight of the monomers M.

Suitable monovinylaromatic monomers Ma include benzene compounds, which bear at least one vinyl or 2-propenyl radical where the benzene ring may additionally carry 1 or 2 C₁-C₁₀-alkyl radicals, particularly C₁-C₄-alkyl radicals. Example of monovinylaromatic monomers include styrene, 2-methylstyrene, 4-methylstyrene, 2-(n-butyl)styrene, 4-(n-butyl)styrene, 4-(n-decyl)styrene and 2-propenylbenzene. In particular, the monovinylaromatic monomers comprise styrene. Especially, the monovinylaromatic monomer Ma is styrene or comprise styrene to an amount of at least 95% by weight, based on the total amount of monovinylaromatic monomers.

Monomers Mb are any ethylenically unsaturated monomer, in particular any monoethylenicallyl unsaturated monomer, which is copolymerizable with the aforementioned monomers Ma. Preferably, the monomers Mb comprise at least one monoethylenically unsaturated acidic monomer Mb1. Examples of monoethylenically unsaturated acidic monomers Mb1 include monoethylenically unsaturated C₃-C₈ monocarboxylic acids, monoethylenically unsaturated C₄-C₈ dicarboxylic acids, C₁-C₄-alkyl monoesters of monoethylenically unsaturated C₄-C₈ dicarboxylic acids and mixtures thereof. The acidic monomers Mb1 may be used in their acidic form or as their salts. Instead of monoethylenically unsaturated C₄-C₈ dicarboxylic acids the anhydrides of monoethylenically unsaturated C₄-C₈ dicarboxylic acids may be used as monomers Mb1.

Suitable monoethylenically unsaturated C₃-C₈ monocarboxylic acids are acrylic acid, methacrylic acid, acryloyloxypropionic acid, methacryloyloxypropionic acid, acryloyloxyacetic acid and methacryloyloxyacetic acid, crotonic acid. The monoethylenically unsaturated C₃-C₈ monocarboxylic acids may also be used in the form of their salts.

Suitable monoethylenically unsaturated C₄-C₈ dicarboxylic acids are selected from maleic acid, fumaric acid, itaconic acid. The monoethylenically unsaturated C₄-C₈ dicarboxylic acids may also be used in the form of their salts, anhydrides, their C₁-C₄-alkyl monoesters esters.

In particular the monomer Mb1 is selected from the group consisting of monoethylenically unsaturated monocarboxylic acids having 3 to 6 carbon atoms and monoethylenically unsaturated dicarboxylic acids having 4 to 8 carbon atoms. Amongst the aforementioned monomers Mb1 monoethylenically unsaturated monocarboxylic acids having 3 to 6 carbon atoms are preferred with preference given to acrylic acid, methacrylic acid, and mixtures thereof. Particular preference is given to methacrylic acid and mixtures thereof with acrylic acid, especially to methacrylic acid. In particular, at least 80% by weight, in particular, at least 95% by weight, based on the total amount of monomers Mb1 is methacrylic acid.

The monomers M may optionally comprise one or more further ethylenically unsaturated monomers Mb2, which are different from the monomer Ma and Mb1.

In accordance with the invention the amount of the monomers Mb2, which are different from the monomer Ma and Mb1, based on the total weight of monomers M, is in the range of from 0 to 29.9% by weight, in particular in the range from 0 to 24% by weight and especially in the range from 0 to 18% by weight or in the range from 0 to 15% by weight, based on the total weight of the monomers M. In a preferred groups of embodiments, the monomer Mb2, if present, is at most 10% by weight, especially at most 5% by weight or even lower, e.g. at most 2% by weight or at most 1% by weight or 0% by weight, based on the total amount of monomers M.

Suitable monomers Mb2, if present, are in particular monoethylenically unsaturated non-ionic monomers, i.e. monoethylenically unsaturated monomers having no cationic, cationogenic, anionic or aniononogenic group. Cationogenic groups are basic groups, which can be converted in to cationic groups by protonation or alkylation. Cationogenic groups are e.g. primary, secondary or tertiary amino groups. Anionogenic groups are acidic groups, which are converted into an anionic group by deprotonation. Examples of anionogogenic groups include the carboxyl group, (COOH), the sulfonatic acid group (SO₃H) and the phosphonic acid group (P(O)(OH)₂). Anionic groups are, in particular, the carboxylate group, i.e. the deprotonated carboxyl group (-COO⁻), but also sulfonate groups (SO₃⁻) or phosphonate groups (PHO₃⁻).

If present, the monomers Mb2 are preferably selected from esters of monoethylenically unsaturated C₃-C₈ monocarboxylic acids with C₁-C₁₀ alkanols, esters of monoethylenically unsaturated C₄-C₈ dicarboxylic acids with C₁-C₁₀ alkanols, esters of vinyl alcohol or allyl alcohol with C₁-C₃₀ monocarboxylic acids, amides and diamides, monoethylenically unsaturated C₃-C₈ monocarboxylic acids with C₁-C₁₀ alkylamines, C₄-C₈ dicarboxylic acids with di-C₁-C₁₀ alkylamines, and mixtures thereof, preferably esters of monoethylenically unsaturated C₃-C₈ monocarboxylic acids with C₁-C₁₀ alkanols, esters of monoethylenically unsaturated C₄-C₈ dicarboxylic acids with C₁-C₁₀ alkanols, and mixtures thereof.

Suitable esters and diesters of monoethylenically unsaturated C₃-C₈ monocarboxylic and C₄-C₈ dicarboxylic acids with C₁-C₃₀ alkanols, more particularly with C₁-C₁₀ alkanols, are especially the esters of monoethylenically unsaturated C₃-C₈ monocarboxylic acids, more particularly the esters of acrylic acid and the esters of methacrylic acid with C₁-C₃₀ alkanols, more particularly with C₁-C₁₀ alkanols, such as methyl (meth)acrylate, methyl ethacrylate, ethyl (meth)acrylate, ethyl ethacrylate, n-propyl (meth)acrylate, isopropyl (meth)acrylate, n-butyl (meth)acrylate, sec-butyl (meth)acrylate, tert-butyl (meth)acrylate, tert-butyl ethacrylate, n-hexyl (meth)acrylate, n-heptyl (meth)acrylate, n-octyl (meth)acrylate, 1,1,3,3-tetramethylbutyl (meth)acrylate, ethylhexyl (meth)acrylate, n-nonyl (meth)acrylate, n-decyl (meth)acrylate, n-undecyl (meth)acrylate, tridecyl (meth)acrylate, myristyl (meth)acrylate, pentadecyl (meth)acrylate, palmityl (meth)acrylate, heptadecyl (meth)acrylate, nonadecyl (meth)acrylate, arachinyl (meth)acrylate, behenyl (meth)acrylate, lignoceryl (meth)acrylate, cerotinyl (meth)acrylate, melissyl (meth)acrylate, palmitoleyl (meth)acrylate, oleyl (meth)acrylate, linolyl (meth)acrylate, linolenyl (meth)acrylate, stearyl (meth)acrylate, and lauryl (meth)acrylate, but also the diesters of monoethylenically unsaturated C₄-C₈ dicarboxylic acids, more particularly the diesters of maleic acid with C₁-C₃₀ alkanols such as dimethyl maleate, diethyl maleate, di(n-propyl) maleate, diisopropyl maleate, di(n-butyl) maleate, di(n-hexyl) maleate, di(1,1,3,3-tetramethylbutyl) maleate, di(n-nonyl) maleate, ditridecyl maleate, dimyristyl maleate, dipentadecyl maleate, dipalmityl maleate, diarachinyl maleate, and mixtures thereof. The term "(meth)acrylate" here embraces both the corresponding ester of acrylic acid and the corresponding ester of methacrylic acid.

Suitable esters of vinyl alcohol and allyl alcohol with C₁-C₃₀ monocarboxylic acids are, for example, vinyl formate, vinyl acetate, vinyl propionate, vinyl butyrate, vinyl laurate, vinyl stearate, vinyl esters of Versatic acid, allyl formate, allyl acetate, allyl propionate, allyl butyrate, allyl laurate, vinyl methacrylate, allyl methacrylate, vinyl acrylate, allyl acrylate, and mixtures thereof.

Suitable amides and diamides of monoethylenically unsaturated C₃-C₈ monocarboxylic and C₄-C₈ dicarboxylic acids with C₁-C₃₀ alkylamines or di-C₁-C₃₀ alkylamines, more particularly with C₁-C₁₀ alkylamines or di-C₁-C₁₀ alkylamines, are especially the amides of acrylic acid and of methacrylic acid with C₁-C₃₀ alkylamines or di-C₁-C₃₀ alkylamines, more particularly with C₁-C₁₀ alkylamines or di-C₁-C₁₀ alkylamines, such as, for example, N-methyl(meth)acrylamide, N-ethyl(meth)acrylamide, N-propyl(meth)acrylamide, N-(n-butyl)(meth)acrylamide, N-(tert-butyl)(meth)acrylamide, N-(n-octyl)(meth)acrylamide, N-(1,1,3,3-tetramethylbutyl)(meth)acrylamide, N-ethylhexyl(meth)acrylamide, N-(n-nonyl)(meth)acrylamide, N-(n-decyl)(meth)acrylamide, N-(n-undecyl)(meth)acrylamide, N-tridecyl(meth)acrylamide, N-myristyl(meth)acrylamide, N-pentadecyl(meth)acrylamide, N-palmityl(meth)acrylamide, N-heptadecyl(meth)acrylamide, N-nonadecyl(meth)acrylamide, N-arachinyl(meth)acrylamide, N-behenyl(meth)acrylamide, N-lignoceryl(meth)acrylamide, N-cerotinyl(meth)acrylamide, N-melissyl(meth)acrylamide, N-palmitoleyl(meth)acrylamide, N-oleyl(meth)acrylamide, N-linolyl(meth)acrylamide, N-linolenyl(meth)acrylamide, N-stearyl(meth)acrylamide, N-lauryl(meth)acrylamide, N,N-dimethyl(meth)acrylamide, N,N-diethyl(meth)acrylamide, but also the diamides and imides of maleic acid with C₁-C₃₀ alkylamines or di-C₁-C₃₀ alkylamines, more particularly with C₁-C₁₀ alkylamines or di-C₁-C₁₀ alkylamines, such as, for example, N,N'-dimethylmaleamide, N,N'-diethylmaleamide, N,N'-dipropylmaleamide, N,N'-di(tert-butyl)maleamide, N,N'-di(n-octyl)maleamide, N,N'-di(n-nonyl)maleamide, N,N'-ditridecylmaleamide, N,N'-dimyristylmaleamide, N,N,N',N'-tetramethylmaleamide, N,N,N',N'-tetraethylmaleamide, and mixtures thereof. The term "(meth)acrylamide" here embraces both the corresponding amide of acrylic acid and the corresponding amide of methacrylic acid.

Further suitable monomers Mb2 are, for example, vinyl halides, vinylidene halides, and mixtures thereof.

Suitable vinyl halides and vinylidene halides are vinyl chloride, vinylidene chloride, vinyl fluoride, vinylidene fluoride, and mixtures thereof.

If present, the monomer Mb2 is more preferably selected from esters of monoethylenically unsaturated C₃-C₈ monocarboxylic acids, especially esters of acrylic acid with C₁-C₆ alkanols, esters of methacrylic acid with C₁-C₆ alkanols, and mixtures thereof. Even more preferably the at least one monomer Mb2 is selected from methyl acrylate, ethyl acrylate, n-butyl acrylate, tert-butyl acrylate, 2-ethylhexyl acrylate, methyl methacrylate, tert-butyl methacrylate, and mixtures thereof.

If present, the monomer Mb2 is especially methyl methacrylate.

Especially, the monomer Mb is methacrylic acid or comprises at least 95% by weight of methacrylic acid, based on the total amount of monomers Mb.

In a preferred embodiment the monomers M comprise
a) 70 to 99.9% by weight, in particular 75 to 99% by weight, especially 80 to 98% by weight or 80 to 95% by weight or 85 to 95% by weight, and most preferably 89 to 93% by weight, based on the total weight of the monomers M, of at least one monomer Ma, and
b.1) 0.1 to 30% by weight, in particular 1 to 25% by weight, especially 2 to 20% by weight or 5 to 20% by weight or 5 to 15% by weight, and most preferably 7 to 11% by weight, based on the total weight of the monomers M, of at least one monomer Mb1, which is preferably selected from the group consisting of monoethylenically unsaturated monocarboxylic acids having 3 to 6 carbon atoms and monoethylenically unsaturated dicarboxylic acids having 4 to 8 carbon atoms and which is particularly selected from the group consisting of methacrylic acid and mixtures thereof with acrylic acid. In particular at least 80% by weight, in particular at least 95% by weight, based on the total amount of monomers Mb1 is methacrylic acid;
b.2) optionally up to 29.9% by weight, in particular 0 to 24% by weight and especially 0 to 18% by weight or 0 to 15% by weight or 0 to 10% by weight or 0 to 5% by weight or 0 to 2% by weight, based on the total weight of the monomers M, based on the total weight of monomers M, one or more further monomers ethylenically unsaturated monomers Mb2, which are different from the monomer Ma and Mb1.

The radical polymerization of the ethylenically usaturated monomers M as defined above takes place in the presence of 80 to 200% by weight, in particular 85 to 170% by weight, especially 95 to 155% by weight, based on the total amount of the monomers M, of a degraded starch.

The term "degraded starch" refers to a starch, whose molecular weight has been reduced by a chemical reaction (degradation), e.g. by hydrolysis or oxidation, and which thus has been converted into a water-soluble form.

In principle, all starches are suitable as a basis for the degraded starch, but preferred starches are those from corn, wheat, rice, tapioca and in particular potatoes. Undegraded starches are virtually insoluble in water and can be converted into a water-soluble form in a known manner by thermal and/or mechanical treatment or by enzymatic, oxidative or acid-catalyzed degradation. Specific examples of starch degradation products obtainable either by oxidative, hydrolytic or enzymatic degradation are: dextrin, such as white and yellow dextrins, maltodextrins, glucose syrups, maltose syrups, hydrolysates with a high content of D-glucose, and maltose and D-glucose and their isomerization product fructose. Of course, monosaccharides and oligosaccharides, such as galactose, mannose, ribose, sucrose raffinose, lactose and trehalose, and products of the degradation of cellulose, for example cellobiose and oligomers thereof, are also suitable as degraded starch component. In a special embodiment the degraded starch is a maltodextrin.

In a preferred embodiment starch which has been degraded enzymatically or by acid catalysis is used. Especially, the degraded starch is an enzymatically degraded starch. Enzymatically or acid catalyzed degraded starches are commercially available and are also known as starch saccharification products. Enzymatically or acid catalyzed degraded starches frequently contain from 0.5 to 5% by weight, in particular from 0.5 to 4% by weight, especially from 0.5 to 3.5% by weight of monosaccharides, namely dextrose. They usually contain from 3 to 40% by weight, in particular 5 to 37% by weight, especially from 5 to 36.5% by weight of disaccharides. They usually contain from and from 2 to 30% by weight, in particular from 5 to 27% by weight, especially from 7 to 23% by weight of triose. They usally contain from 30 to 94.5% by weight, preferably 35 to 89.5% by weight, in particular from 30 to 87.5% by weight of higher oligodextrose.

Maltodextrin is typically composed of a mixture of chains that vary from 3 to 20 glucose units long. Maltodextrins are classified by DE (dextrose equivalent). The DE is a measure of the amount of reducing sugars present in a sugar product, expressed as percentage on a dry basis relative to dextrose. Usually, the DE is determined in accordance to the method of Lane and Eynon and expressed as % inverted sugar or in accordance to the method of Luff-Schoorl and expressed in meq glucose/g. The respective methods are described in the Official Journal of the European Communities No. L/239/24-52 of 22.09.1979 (79/78 6/EEC), methods 6 (Luff-Schoorl) and 7 or 8 (Lane Eynon).

For the purpose of the invention, degraded starches, in particular maltodextrins, are preferred, which have a DE according to Lane and Eynon in the range from 2 and 40%, preferably in the range from 15 to 40% and especially in the range from 20 to 40% or a DE according to Luff-Schoorl in the range from 2.0 to 10 meq/g, in particular in the range from 2.8 to 7.5 and especially in the range from 3.0 to 7.0 (detertmined by Luff-Schoorl).

For the purpose of the invention degraded starches, in particular maltodextrins, are preferred, whose 50% by weight aqueous solution have a viscosity of at least 30 mPas, preferably at least 50 mPas, e.g. in the range of 30 to 300 mPas, in particular in the range from 50 to 280 mPas, as determined in a rotational viscometer at 20°C and 1 rpm.

Preferably, the degraded starch has a number average molecular weight in the range from 300 to 2000 g/mol, in particular in the range from 350 to 1500 Dalton and especially in the range from 350 to 1000 g/mol as determined by gel permeation chromatography, or as calculated from the dextrose equivalent according to Luff-Schoorl.

Preferably, the degraded starch has a weight average molecular weight in the range from 800 to 30000 Dalton, in particular in the range from 1000 to 10000 Dalton as determined by gel permeation chromatography.

Gel permeation chromatography is carried of the degraded starch is carried out by analogy to standard methods disclosed in the art using e.g. polyester copolymers as stationary phase, dimethylacetamide + 0.5% LiBr as eluent and polymethylmethacrylate standards (molar mass range 800-2200000 g/mol). For more details reference is made to the examples.

Preferably, the degraded starch has a number average molecular weight in the range from 300 to 2000 Dalton, in particular in the range from 350 to 1500 Dalton and especially in the range from 350 to 1000 g/mol as determined by osmometry. The number average molecular weights given here refer to the values as determined by the method described by Y. Rong, M. Sillick and C M. Gregson in Journal of Food Science 2009, 74 (1), pp C33-040 ("Determination Of Dextrose Equivalent Value And Number Average Molecular Weight Of Maltodextrin By Osmometry").

Examples of commercially available degraded starches are the starch products available under the following brand names: C*Dry GL 01924, C*Dry MD 01915, C*Dry A 01318, C*Dry A 01321 ^{®}, C*Dry GL 01934 ^{®}, and C*Dry GL 01932 all of Cargill and Roclys C 1967 S of Roquette.

In an especially preferred embodiment the aqueous polymer dispersion is obtainable by radical aqueous emulsion polymerization of monomers M comprise
a) 80 to 98% by weight or 80 to 95% by weight or 85 to 95% by weight, and most preferably 89 to 93% by weight, based on the total weight of the monomers M, of at least one monomer Ma, which comprises at least 95% of styrene, and
b.1) 2 to 20% by weight or 5 to 20% by weight or 5 to 15% by weight, and most preferably 7 to 11% by weight, based on the total weight of the monomers M, of at least one monomer Mb1, which is preferably selected from the group consisting of monoethylenically unsaturated monocarboxylic acids having 3 to 6 carbon atoms and monoethylenically unsaturated dicarboxylic acids having 4 to 8 carbon atoms and which is particularly selected from the group consisting of methacrylic acid and mixtures thereof with acrylic acid. In particular at least 80% by weight, in particular at least 95% by weight, based on the total amount of monomers Mb1 is methacrylic acid;
b.2) optionally 0 to 5% by weight or 0 to 2% by weight, based on the total weight of the monomers M, based on the total weight of monomers M, one or more further monomers ethylenically unsaturated monomers Mb2, which are different from the monomer Ma and Mb1,
in the presence of 85 to 170% by weight, especially 95 to 155% by weight, based on the total amount of the monomers M, of a maltodextrin, which has a DE according to Lane Eynon in the range from 15 to 40% and especially in the range from 20 to 40% or a DE according to Luff-Schoorl in the range from 2.8 to 7.5 and especially in the range from 3.0 to 7.0 (detertmined by Luff-Schoorl) and/or which has a number average molecular weight in the range from 350 to 1000 g/mol as determined by gel permeation chromatography, or as calculated from the dextrose equivalent according to Luff-Schoorl and/or which has a number average molecular weight in the range from 350 to 1000 Dalton as determined by osmometry.

Frequently, the dispersed particles of the aqueous polymer dispersions have a volume median particle diameter (also termed as D(v 0.5)value) as determined by static light scattering (SLS) of at least 150 nm, in particular at least 170 nm, more preferably at least 180 nm and especially at least 190 nm or at least 200 nm. In particular the D(v 0.5) value is in the range from 150 to 1000 nm, more preferably in the range from 170 to 900 nm or in the range from 180 to 800 nm and especially in the range from 190 to 750 nm or in the range from 200 to 700 nm. Frequently the aqueous polymer dispersions have a particle size distribution, wherein the D(v, 0.1) value is in the range of 50 to 200 nm, in particular in the range from 80 to 180 nm. Frequently the aqueous polymer dispersions have a particle size distribution, wherein the D(v, 0.9) value is at least 250 nm, preferably at least 300 nm, in particular at least 350 nm, especially at least 400 nm and e.g. in the range of 250 to 2500 nm, preferablly in the range from 300 to 2500 nm or in the range from 350 to 2000 nm. Frequently the aqueous polymer dispersions have a particle size distribution, wherein the volume or mass moment mean, also termed D[4,3] value is at least 220 nm, preferably at least 250 nm, in particular at least 300 nm, especially at least 350 nm, e.g. in the range from 220 to 1200 nm, preferably in the range from 250 to 1000 nm, in particular in the range from 300 to 900 nm and especially in the range from 350 to 800 nm.

The particle size distribution of the dispersed particles in the aqueous polymer dispersion may be monomodal or polymodal, with preference given to the latter. In particular, it is beneficial with regard to the opacifying, if at least 15% by volume, in particular from 15 to 60% by volume of the polymer particles have a particle size of at least 300 nm, in particular of at least 350 nm, especially of at least 400 nm, e.g. in the range from 300 to 2500 nm, in particular in the range from 350 to 2000 nm, especially in the range from 400 to 1900 nm.

Here and throughout the specification the terms "particle size" and "particle diameter" are used synonymously and relate to the particle size as determined by static light scattering of the diluted aqueous dispersion in accordance with ISO 13320:2009. This also applies to the particle size distribution which can be characterized e.g. by the following values D[v, 0.1], D[v, 0.5], D[v, 0.9], D[4,3] and D[3,2]. In the context, the value D[v, 0.1] means that 10 Vol.-% of the particles in the probe have a particle size below the value given as D[v, 0.1] value. Correspondingly the value D[v, 0.5] means that 50 Vol.-% of the particles in the probe have a particle size below the value given as D[v, 0.5] value and the value D[v, 0.9] means that 90 Vol.-% of the particles in the probe have a particle size below the value given as D[v, 0.9] value. The value D[4,3] is the volume or mass moment mean, also termed as De Broucker mean. The value D[3, 2] is the surface area moment mean, also termed the Sauter mean diameter.

The solids content of the aqueous polymer dispersions is usually in the range from 30.0 to 60.0% by weight and in particular in the range from 35 to 50% by weight, based on the total weight of the aqueous polymer dispersions.

As the polymer contained in the aqueous polymer dispersions of the invention contains a considerable amount of degraded starch, the polymers are biodegradable or at least can be eliminated from the sewage plant effluent together with the sewage sludge. It is particularly noteworthy that the aqueous polymer dispersions of the invention contain considerable amount of degraded starch in addition to the polymer formed from the polymerized monomers M, namely 44 to 67% by weight, based on the total solids contained in the polymer dispersion, of a degraded starch, but still an opazifing effect which is at least comparable or even better than the opacifing effect of an aqueous polymer dispersion, wherein the polymer is exclusively formed of ethylenically unsaturated monomers and which does not contain a degraded starch.

The aqueous polymer dispersion used according to the invention is obtainable by radical aqueous emulsion polymerization of
i) ethylenically unsaturated monomers M comprising
   a) at least 80% by weight, in particular at least 85% by weight, especially at least 89% by weight, based on the total weight of monomers M, of at least one monomer Ma, selected from the group consisting of monovinylaromatic monomers, and optionally
   b) up to 20% by weight, in particular up to 15% by weight, especially up to 11% by weight, based on the total weight of monomers M, of one or more ethylenically unsaturated monomers Mb, which are different from the monomer Ma;
   in the presence of
ii) 80 to 200%, in particular 85 to 170% by weight, especially 95 to 155% by weight, based on the total amount of monomers M, of a degraded starch as defined herein.

With regard to the monomers M suitable as component i), especially monomers Ma suitable as component a) and monomers Mb suitable as component b), reference is made to the details above. Likewise, with regard to the degraded starch as component ii), in particular, its number average molecular weight and its DE value according to Lane and Eynon, reference is made to the details above.

In a preferred embodiment, the aqueous polymer dispersion used according to the invention is obtainable by radical emulsion polymerization of monomers M, where the monomers M comprise
a) 80 to 99.9% by weight or 80 to 95% by weight or 85 to 95% by weight, and most preferably 89 to 93% by weight, based on the total weight of the monomers M, of at least one monomer Ma, and
b.1) 0.1 to 20% by weight or 5 to 20% by weight or 5 to 15% by weight, and most preferably 7 to 11% by weight, based on the total weight of the monomers M, of at least one monomer Mb1, selected from the group consisting of monoethylenically unsaturated monocarboxylic acids having 3 to 6 carbon atoms and monoethylenically unsaturated dicarboxylic acids having 4 to 6 carbon atoms.

In a preferred group of embodiments the majority of the monomers M to be polymerized are fed to an aqueous solution of the degraded starch under polymerization conditions. In this group of embodiments in particular at least 70% by weight, especially at least 90% by weight, of the monomers M to be polymerized, based on the total amount of monomers M, are fed to an aqueous solution of the degraded starch under polymerization conditions.

In this preferred group of embodiments, in particular an initial portion of the monomer M, which essentially or completely consists of monovinylaromatic monomers Ma, is mixed with an aqueous solution of the degraded starch and polymerized to obtain an initial polymerization mixture and then the remainder of the monomers M are added to the initial polymerization mixture and polymerized. The initial polymerization mixture is an aqueous polymer dispersion, wherein the polymer particles contain the polymerized monomers M of the initial portion and the degraded starch.

In this context, the term "essentially consists of" means that the relative amount of the monovinylaromatic monomers Ma is at least 99% by weight, in particular at least 99.5% by weight and especially at least 99.9% by weight, based on the total amount of monomers in that initial portion of monomers M.

In this preferred group of embodiments, the initial portion may be added all at once to the aqueous solution of the degraded starch and then polymerization is started. However it is preferred that at least the majority, i.e. more than 50% by weight, in particular at least 70% by weight, especially at least 90% by weight of the initial feed of monomer M, are fed to the aqueous solution of the degraded starch under polymerization conditions to obtain the initial polymer dispersion.

Consequently, the preferred group of embodiments relates to the use according to the invention of the polymer dispersions as defined above, which can in particular be prepared by a process comprising the steps:
a) providing an initial feed comprising at least a part of the monomer component M to be polymerized, where this initial part essentially or completely consists of monovinylaromatic monomers Ma,
b) feeding at least the majority, i.e. more than 50% by weight, in particular at least 70% by weight, especially at least 90% by weight of the initial feed of monomer M to an aqueous solution of the degraded starch under polymerization conditions to obtain the initial polymer dispersion, and
c) feeding the remainder of the monomers M to the initial polymerization product under polymerization conditions to obtain the aqueous polymer dispersion of the present invention.

The term "polymerization conditions" is generally understood to mean those temperatures and pressures under which the free-radically initiated aqueous emulsion polymerization proceeds at sufficient polymerization rate. They depend particularly on the free-radical initiator used. Advantageously, the type and amount of the free-radical initiator, polymerization temperature and polymerization pressure are selected such that a sufficient amount of initiating radicals is always present to initiate or to maintain the polymerization reaction.

The amount of the initial portion is in particular in the range from 50 to 95% by weight, especially in the range from 60 to 90% by weight, based on the total of the monomers to be polymerized. Consequently, the remainder the monomers M is in the range from 5 to 50% by weight, especially in the range from 10 to 40% by weight, based on the total of the monomers to be polymerized.

In this preferred group of embodiments, preferably at least 70% by weight, especially at least 90% by weight, of said initial portion of the monomers M are fed to the aqueous solution of the degraded starch under polymerization conditions. Said initial portion is preferably fed to the aqueous solution of the degraded starch over an extended period of time, which may preferably last from 20 minutes to 8 h, in particular from 30 minutes to 5 h.

In this preferred group of embodiments, the remainder of the monomers may be added all at once to the initial polymerization mixture. However a portion or all of the remainder portion of monomers M, in particular the majority, i.e. at least 50% by weight, in particular at least 70% by weight, especially at least 90% by weight, of said remainder portion of the monomers M are fed to the polymerization mixture under polymerization conditions. Said remainder portion is preferably fed to the aqueous initial polymer dispersion over an extended period of time, which may preferably last from 10 minutes to 5 h, in particular from 20 minutes to 4 h.

The addition of the remainder of the monomers to the initial polymerization mixture is usually not started before the initial portion has been completely fed to the polymerization reaction. It may also be possible to interrupt the polymerization at the stage of the initial polymerization mixture, then add a portion or all of the remainder of monomers and then restart the polymerization.

The monomers initially fed to the aqueous solution of the degraded starch may be fed as such or preferably as an aqueous emulsion. Likewise, the remainder of the monomers fed to the polymerization mixture may be fed as such or as an aqueous emulsion.

In this preferred group of embodiments a small portion of the monomers M in particular at most 20% by weight, especially at most 10% by weight, of the monomers M of the initial portion may be charged into the aqueous solution of the degraded starch all at once before polymerization is started and then the mixture is subjected to polymerization conditions and the remainder of the monomers M of the initial portion is fed to the polymerization reaction under polymerization conditions.

In a preferred embodiment the remainder of the monomers M is a mixture of the monomer Ma and at least one monomer Mb, in particular a mixture of at least one monomer Ma and at least one monomer Mb1 and optionally one or more monomers Mb2. In a special embodiment the remainder of the monomers M is a mixture styrene and a monomer Mb1, which is especially methacrylic acid.

The conditions required for the performance of the radical aqueous emulsion polymerization of the monomers M in the presence of the degraded starch are sufficiently familiar to those skilled in the art, for example from the prior art cited at the outset and from "Emulsionspolymerisation" [Emulsion Polymerization] in Encyclopedia of Polymer Science and Engineering, vol. 8, pages 659 ff. (1987); D. C. Blackley, in High Polymer Latices, vol. 1, pages 35 ff. (1966); H. Warson, The Applications of Synthetic Resin Emulsions, chapter 5, pages 246 ff. (1972); D. Diederich, Chemie in unserer Zeit 24, pages 135 to 142 (1990); Emulsion Polymerisation, Interscience Publishers, New York (1965); DE-A 40 03 422 and Dispersionen synthetischer Hochpolymerer [Dispersions of Synthetic High Polymers], F. Hölscher, Springer-Verlag, Berlin (1969)].

Preference is given to operation in the absence of oxygen, preferably in a stream of nitrogen. For the polymerization method any customary apparatus can be used, examples thereof including stirred tanks, stirred tank cascades, autoclaves and tubular reactors.

The radical aqueous emulsion polymerization of the invention is usually conducted at temperatures in the range from 0 to 170°C. Temperatures employed are frequently in the range from 40 to 140°C, in particular in the range from 50 to 120°C and especially in the range from 60 to 110°C.

The radical aqueous emulsion polymerization is triggered by means of a free-radical polymerization initiator (free-radical initiator). These may in principle be peroxides or azo compounds and so-called redox initiator systems. Peroxides used may, in principle, be inorganic peroxides, such as hydrogen peroxide or peroxodisulfates, such as the mono- or di-alkali metal or ammonium salts of peroxodisulfuric acid, for example the mono- and disodium, -potassium or ammonium salts, or organic peroxides such as alkyl hydroperoxides, for example tert-butyl hydroperoxide, p-menthyl hydroperoxide or cumyl hydroperoxide, and also dialkyl or diaryl peroxides, such as di-tert-butyl or dicumyl peroxide. Azo compounds used are essentially 2,2'-azobis(isobutyronitrile), 2,2'-azobis(2,4-dimethylvaleronitrile) and 2,2' azobis(amidinopropyl) dihydrochloride (AIBA, corresponds to V-50 from Wako Chemicals).

Preferably, a redox initiator is used for initiating the polymerization. Redox initiators usually comprise an oxidizing agent and a reducing agent or an oxidizing agent and a transition metal, which catalyzes the decomposition of the oxidizing agent. Said redox initiator is preferably water-soluble. Suitable oxidizing agents for redox initiator systems are essentially the peroxides specified above. Corresponding reducing agents which may be used are sulfur compounds with a low oxidation state, such as alkali metal sulfites, for example potassium and/or sodium sulfite, alkali metal hydrogensulfites, for example potassium and/or sodium hydrogensulfite, alkali metal metabisulfites, for example potassium and/or sodium metabisulfite, aldehyde sulfoxylates, in particular formaldehyde sulfoxylates, for example potassium and/or sodium formaldehyde sulfoxylate, ketone sulfoxylates, in particular bis(C₁-C₄-alkyl)ketone sulfoxylates, such as acetone sulfoxylate and methylethylketone sulfoxylates and the respective salts thereof, in particular the alkalimetal salts thereof, alkali metal salts, specifically potassium and/or sodium salts of aliphatic sulfinic acids and alkali metal hydrogensulfites, for example potassium and/or sodium hydrogensulfite, ene diols, such as dihydroxymaleic acid, benzoin and/or ascorbic acid, and reducing saccharides, such as sorbose, glucose, fructose and/or dihydroxyacetone.

Suitable transition metals include but are not limited to salts of iron, cobalt, cerium, nickel, copper, vanadium and manganese, in particular iron(II) salts, cobalt(II) salts, cerium(III) salts, cerium(IV) salts, nickel(II) salts and copper(I) salts. Preferred transition metal salts include, for example, iron(II) sulfate, iron(II) ammonium sulfate (Mohr's salt), iron(ll) phosphate, cobalt(II) chloride, cerium(III) nitrate, cerium(IV) sulfate, ammonium cerium(IV) sulfate, ammonium cerium(IV) nitrate, nickel(II) sulfate and copper(I) chloride and complexes of iron(II) salts, cobalt(II) salts, nickel(II) salts with a chelating agent such as ethylenediaminetetraacetic acid (EDTA), Diethylenetriaminepentacetic acid (DPTA), methylglycinediacetic acid (MGDA) and N,N-bis(carboxymethyl)glutamic acid (GLDA).

The free-radical initiator are usually employed in an amount of 0.05 to 15% by weight, in particular in an amount from 0.1 to 10% by weight especially in an amount from 0.5 to 8 wt%, based on the monomers M to be polymerized. In the case of a multicomponent initiator systems (e.g., redox initiator systems), the weight figures above are based on the total sum of the components.

If the redox initiator comprises a transition metal salt, the transition metal salt is frequently used in an amount of 0.1 ppm to 1000 ppm, in particular from 1 ppm to 500 ppm, especially 2 ppm to 200 ppm, based on the weight of monomers M to be polymerized, or on monomers M to be polymerized in any one stage.

In a preferred embodiment a radical polymerization initiator is used, which comprises or consists of a peroxide, which is in particular selected from the group consisting of H₂O₂ and peroxodisulfate and mixtures thereof, and a transition metal salt, in particular an iron(ll) salt, a cerium(III) salt or a cerium(IV) salt. Such combinations usually comprise from 0.5 to 10% of hydrogen peroxide and 0.1 ppm to 1000 ppm, in particular from 1 ppm to 500 ppm, especially 2 ppm to 200 ppm, of the transition metal salt, in each case based on the total amount of monomers M.

The emulsion polymerization of the monomers M as defined above is effected in an aqueous medium in the presence of a degraded starch, as defined above. The monomers M can be polymerized by the radical emulsion polymerization method, both in the feed procedure and in the batch procedure as described above. If the redox initiator comprises a transition metal salt, preferably an aqueous solution of the degraded starch and the transition metal salt is initially charged and the monomers are added continuously or batch-wise, either separately or as a mixture and separately therefrom, to that part of the redox initiator which has oxidizing activity, preferably hydrogen peroxide. A gradient procedure which is disclosed in WO 02/14393 can also be used for the preparation of the polymer dispersions of the present invention. The initiator can also be added in stages, and/or the rate of initiator addition varied over time.

When the reaction mixture is initially polymerized at a lower temperature range and then fully polymerized at a higher temperature, it may be advantageous to use two or more different initiators or initiator systems that decompose at different temperatures, so that an adequate concentration of radicals is available within every temperature interval, or to use a redox initiator system wherein the peroxide-containing component is initially activated by a co-initiator at a low temperature and thermally decomposes at a higher temperature without a continued need for co-initiator.

Preferably, the monomers M are polymerized in the presence of at least one emulsifier E. This emulsifier serves for stabilizing the emulsion of the monomers and the polymer particles of the polymer dispersion. In contrast to the degraded emulsifiers typically have lower molecular weights, in particular number average molecular weights of below 1000 g/mol. The emulsifier E may be anionic or nonionic or mixtures of non-ionic and anionic emulsifiers.

Anionic emulsifiers usually bear at least one anionic group, which is selected from phosphate, phosphonate, sulfate and sulfonate groups. The anionic emulsifiers, which bear at least one anionic group, are typically used in the form of their alkali metal salts, especially of their sodium salts or in the form of their ammonium salts.

Preferred anionic are anionic emulsifiers, which bear at least one sulfate or sulfonate group. Likewise, anionic emulsifiers, which bear at least one phosphate or phosphonate group may be used, either as sole anionic emulsifiers or in combination with one or more anionic emulsifiers, which bear at least one sulfate or sulfonate group.

Examples of anionic emulsifiers, which bear at least one sulfate or sulfonate group, are, for example,
- the salts, especially the alkali metal and ammonium salts, of alkyl sulfates, especially of C₈-C₂₂-alkyl sulfates,
- the salts, especially the alkali metal and ammonium salts, of sulfuric monoesters of C₂-C₃-alkoxylated alkanols, especially of sulfuric monoesters of C₂-C₃-alkoxylated C₈-C₂₂-alkanols, preferably having an C₂-C₃-alkoxylation level (AO level) in the range from 2 to 40,
- the salts, especially the alkali metal and ammonium salts, of sulfuric monoesters of C₂-C₃-alkoxylated alkylphenols, especially of sulfuric monoesters of C₂-C₃-alkoxylated C₄-C₁₈-alkylphenols (AO level preferably 3 to 40),
- the salts, especially the alkali metal and ammonium salts, of alkylsulfonic acids, especially of C₈-C₂₂-alkylsulfonic acids,
- the salts, especially the alkali metal and ammonium salts, of dialkyl esters, especially di-C₄-C₁₈-alkyl esters of sulfosuccinic acid,
- the salts, especially the alkali metal and ammonium salts, of alkylbenzenesulfonic acids, especially of C₄-C₂₂-alkylbenzenesulfonic acids, and
- the salts, especially the alkali metal and ammonium salts, of mono- or disulfonated, alkyl-substituted diphenyl ethers, for example of bis(phenylsulfonic acid) ethers bearing a C₄-C₂₄-alkyl group on one or both aromatic rings. The latter are common knowledge, for example from US-A-4,269,749, and are commercially available, for example as Dowfax^{®} 2A1 (Dow Chemical Company).

The above-mentioned term C₂-C₃-alkoxylated means that the compounds are ethoxylated, propoxylated or co-ethoxylated/propoxylated. In other words, the term C₂-C₃-alkoxylated means that the respective compounds are obtained by a process which introduces a polyethylenoxide group, a polypropyleneoxide group or a poly(ethyleneoxide-co-propyleneoxide) group.

Examples of anionic emulsifiers, which bear a phosphate or phosphonate group, include, but are not limited to the following salts are selected from the following groups:
- the salts, especially the alkali metal and ammonium salts, of mono- and dialkyl phosphates, especially C₈-C₂₂-alkyl phosphates,
- the salts, especially the alkali metal and ammonium salts, of phosphoric monoesters of C₂-C₃-alkoxylated alkanols, preferably having an alkoxylation level in the range from 2 to 40, especially in the range from 3 to 30, for example phosphoric monoesters of ethoxylated C₈-C₂₂-alkanols, preferably having an ethoxylation level (EO level) in the range from 2 to 40, phosphoric monoesters of propoxylated C₈-C₂₂-alkanols, preferably having a propoxylation level (PO level) in the range from 2 to 40, and phosphoric monoesters of ethoxylated-co-propoxylated C₈-C₂₂-alkanols, preferably having an ethoxylation level (EO level) in the range from 1 to 20 and a propoxylation level of 1 to 20,
- the salts, especially the alkali metal and ammonium salts, of phosphoric monoesters of C₂-C₃-alkoxylated alkylphenols, especially phosphoric monoesters of C₂-C₃-alkoxylated C₄-C₁₈-alkylphenols (AO level preferably 3 to 40),
- the salts, especially the alkali metal and ammonium salts, of alkylphosphonic acids, especially C₈-C₂₂-alkylphosphonic acids and
- the salts, especially the alkali metal and ammonium salts, of alkylben-zenephosphonic acids, especially C₄-C₂₂-alkylbenzenephosphonic acids.

Preferred anionic emulsifiers are selected from the following groups:
- the salts, especially the alkali metal and ammonium salts, of alkyl sulfates, especially of C₈-C₂₂-alkyl sulfates,
- the salts, especially the alkali metal salts, of sulfuric monoesters of C₂-C₃-alkoxylated alkanols, especially of sulfuric monoesters of C₂-C₃-alkoxylated C₈-C₂₂-alkanols, preferably having an AO level in the range from 2 to 40,
- of sulfuric monoesters of C₂-C₃-alkoxylated alkylphenols, especially of sulfuric monoesters of C₂-C₃-alkoxylated C₄-C₁₈-alkylphenols (AO level preferably 3 to 40),
- of alkylbenzenesulfonic acids, especially of C₄-C₂₂-alkylbenzenesulfonic acids, and
- of mono- or disulfonated, alkyl-substituted diphenyl ethers, for example of bis(phenylsulfonic acid) ethers bearing a C₄-C₂₄-alkyl group on one or both aromatic rings.

Suitable emulsifiers may also be nonionic emulsifiers. Suitable nonionic emulsifiers are e.g. araliphatic or aliphatic nonionic emulsifiers, for example
- C₂-C₃-alkoxylated mono-, di- and trialkylphenols (AO level: 3 to 50, alkyl radical: C₄-C₁₀),
- C₂-C₃-alkyoxlates, thus ethoxylates, propoxylates or ethoxylate-co-propoxylates, of long-chain alcohols (AO level: 3 to 100, alkyl radical: C₈-C₃₆), and
- polyethylene oxide/polypropylene oxide homo- and copolymers. These may comprise the alkylene oxide units copolymerized in random distribution or in the form of blocks.

Amongst nonionic emulsifiers, preference is given to C₂-C₃-alkoxylated of long-chain alkanols, in particular to those where the alkyl radical C₈-C₃₀ having a mean alkoxylation level of 5 to 100 and, among these, particular preference to those having a linear C₁₂-C₂₀ alkyl radical and a mean alkoxylation level of 10 to 50, and also to C₂-C₃-alkoxylated monoalkylphenols. Emulsifiers are frequently used in an amount of 0.05 to 10%, based on the monomers M to be polymerized.

The polymerization is usually carried out in the absence of oxygen, preferably in an inert gas atmosphere, for example under nitrogen. During the polymerization, thorough mixing of the components should be ensured. Thus, the reaction mixture is preferably stirred for the entire duration of the polymerization and of any subsequent postpolymerization.

The polymerization is usually carried out at a pH of from 2 to 9, preferably in the weakly acidic range at a pH of from 3 to 5.5. The pH can be adjusted to the desired value before or during the polymerization using conventional acids, such as hydrochloric acid, sulfuric acid or acetic acid, or using bases, such as sodium hydroxide solution, potassium hydroxide solution, ammonia, ammonium carbonate, etc. The dispersions are preferably adjusted to a pH of from 5 to 7 with sodium hydroxide solution, potassium hydroxide solution or ammonia after the end of the polymerization.

In order to remove the remaining monomers from the polymer dispersions as substantially as possible, a postpolymerization is expediently carried out. For this purpose, an initiator from the group consisting of hydrogen peroxide, peroxides, hydroperoxides and/or azo initiators is added to the polymer dispersions after the end of the main polymerization. The combination of the initiators with suitable reducing agents, such as, for example, ascorbic acid or sodium bisulfite, is also possible. Oil-soluble initiators which are sparingly soluble in water may also be used, for example conventional organic peroxides, such as dibenzoyl peroxide, di-tert-butyl peroxide, tert-butyl hydroperoxide, cumyl hydroperoxide or biscyclohexyl peroxydicarbonate.

For the postpolymerization, the reaction mixture is heated, for example, to a temperature which corresponds to the temperature at which the main polymerization was carried out or which is up to 20°C, preferably up to 10°C, higher. The main polymerization is complete when the polymerization initiator has been consumed or the monomer conversion is, for example, at least 98%, preferably at least 99.5%. tert-Butyl hydroperoxide is preferably used for the postpolymerization. The postpolymerization is carried out, for example, in a temperature range from 35 to 100°C, preferably from 45 to 95°C.

After the end of the polymerization, a complexing agent for heavy metal ions can be added to the polymer dispersions in an amount such that all heavy metal ions are bound in complexed form.

The invention relates to the use of the polymer dispersions described herein as an opacifier in liquid formulations. In particular, the invention relates to the use of these polymer dispersions as an opacifier in liquid detergent formulation and liquid cosmetic preparations. Such liquid formulations frequently comprise from 0.1 to 10% by weight, in particular from 0.5 to 5% by weight, especially 1 to 3% by weight, based on the total weight of the liquid formulation and calculated as polymer solids, of at least one polymer dispersion described herein.

The invention relates in particular to liquid detergent formulations, which contain at least one polymer dispersion described herein. More particularly, the invention relates to liquid detergent formulations, which are selected from the group consisting of liquid detergent formulations for personal care, washing formulations, cleaning formulations and dishwashing formulations, especially liquid laundry detergent formulations, dishwashing formulations, cleaning formulations, including liquid hard surface cleaning formulations, multipurpose cleaners, kitchen cleaners, manual washing and cleaning formulations, bathroom cleaners, furniture cleaners or any kind of pre-dosed liquid detergent formulation. In particular, such a liquid detergent formulation comprises from 0.5 to 5% by weight, especially from 1 to 3% by weight, calculated as polymer solids and based on the total weight of the liquid formulation, of at least one polymer dispersion described herein.

Further, the invention also relates to cosmetic preparations like hair shampoos, hair lotions, foam baths, shower baths, oral and dental care products, creams, gels and lotions. In particular, such a cosmetic preparation comprises from 0.5 to 5% by weight, especially from 1 to 3% by weight, calculated as polymer solids and based on the total weight of the liquid formulation, of at least one polymer dispersion described herein.

### Liquid detergent formulation: washing, cleaning and dishwashing compositions

The polymer dispersions described herein are particularly suitable for use in liquid washing and cleaning formulations, in dishwashing formulations, in rinse aids and in pre-dosed liquid formulations.

Washing formulations in the context of the present invention are understood to mean those formulations which are used for cleaning flexible non-living materials having high absorbency, for example materials having a textile character, whereas cleaning compositions in the context of the present invention are understood to mean those compositions which are used for cleaning materials having a closed surface, i.e. having a surface which has only few and small pores, if any, and consequently has zero or only low absorbency.

Examples of flexible materials having high absorbency are those which comprise or consist of natural, synthetic or semisynthetic fiber materials, and which accordingly generally have at least some textile character. The fibrous materials or materials consisting of fibers may in principle be present in any form which occurs in use or in manufacture and processing. For example, fibers may be present in unordered form in the form of staple or aggregate, in ordered form in the form of fibers, yarns, threads, or in the form of three-dimensional structures such as nonwovens, lodens or felt, wovens, knits, in all conceivable binding types. The fibers may be raw fibers or fibers in any desired stages of processing. Examples are natural protein or cellulose fibers, such as wool, silk, cotton, sisal, hemp or coconut fibers, or synthetic fibers, for example polyester, polyamide or polyacrylonitrile fibers.

Examples of materials having only few and small pores, if any, and having zero or only low absorbency are metal, glass, enamel or ceramic. Typical objects made of these materials are, for example, metallic sinks, cutlery, glass and porcelain dishware, bathtubs, washbasins, tiles, flags, cured synthetic resins, for example decorative melamine resin surfaces on kitchen furniture or painted metal surfaces, for example refrigerators and car bodies, printed circuit boards, microchips, sealed or painted woods, e.g. parquet or wall cladding, window frames, doors, plastics coverings such as floor coverings made of PVC or hard rubber, or rigid or flexible foams having substantially closed surfaces.

Examples of cleaning formulations comprising a polymer dispersion described herein include washing and cleaning formulations, dishwashing formulations such as manual dishwashing formulations or machine dishwashing formulations (= a dishwashing formulations for machine dishwashers), metal degreasers, glass cleaners, floor cleaners, all-purpose cleaners, high-pressure cleaners, neutral cleaners, alkaline cleaners, acidic cleaners, spray degreasers, dairy cleaners, commercial kitchen cleaners, machinery cleaners in industry, especially the chemical industry, cleaners for carwashing and also domestic all-purpose cleaners.

A preferred group of embodiments of liquid detergent formulations are washing compositions, cleaning compositions and dishwashing compositions.

In these liquid detergent formulations, the polymer dispersions of the present invention show excellent action as a opacifier.

The liquid detergent formulations of the present invention preferably comprise the following constituents:
(a) at least one polymer dispersion described in the context of the present invention;
(b) at least one surface-active ingredient, also termed surfactant;
(c) water;
(d) optionally one or more enzymes;
(e) optionally one or more builders (also referred to as sequestrant, builder material, complexing agent, chelator, chelating agent or softener);
(f) optionally one or more further additives other than a), b), d) and e), such as bases, corrosion inhibitors, defoamers, dyes, fragrances, fillers, thickeners, solubilizers, organic solvents, electrolytes, pH modifiers, perfume carriers, fluorescers, hydrotropes, antiredeposition agents, optical brighteners, graying inhibitors, shrink inhibitors, grease inhibitors, dye transfer inhibitors, active antimicrobial ingredients, antioxidants, corrosion inhibitors, antistats, ironing aids, hydrophobizing and impregnating agents, swell and antislip agents and UV absorbers.

Preferably, the liquid detergent formulations of the present invention comprise:
(a) 0.1 to 10% by weight, in particular 0.5 to 5% by weight of at least one polymer dispersion described herein, calculated as polymer solids,
(b) 0.2 to 70% by weight, in particular 0.4 to 60% by weight, of at least one surfactant, which is preferably selected from the group consisting of nonionic surfactants, anionic surfactants, cationic surfactants amphoteric surfactants and mixtures thereof,
(c) 5 to 99.6% by weight, in particular 10 to 98% by weight of water,
   and optionally
(d) 0 to 10% by weight, in particular 0 to 5% by weight of one or more enzymes,
(e) 0 to 10% by weight of one or more builders and/or cobuilders,
(f) 0 to 60% by weight of one or other further additives mentioned above,
where the amount given for components (a) to (g) are based on the total weight of the formulation.

### Component a)

With regard to polymer dispersions suitable and preferred as component a), reference is made to the details above.

### Component b)

Suitable surfactants are anionic surfactants, nonionic surfactants, cationic surfactants, amphoteric surfactants and mixtures thereof.

Typical examples of anionic surfactants are soaps, alkylsulfonates, alkylbenzenesulfonates, olefinsulfonates, alkyl ether sulfonates, glycerol ether sulfonates, methyl ester sulfonates, sulfo fatty acids, alkylsulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, such as, for example, acylglutamates and acylaspartates, and also acyllactylates, acyltartrates, alkyl oligoglucosidesulfates, alkyl glucose carboxylates, protein fatty acid condensates and alkyl (ether) phosphates.

Suitable soaps are e.g. alkali metal, alkaline earth metal and ammonium salts of fatty acids, such as potassium stearate.

Suitable olefinsulfonates are obtained e.g. by the addition reaction of SO₃ onto olefins of the formula R³-CH=CH-R⁴ and subsequent hydrolysis and neutralization, where R³ and R⁴, independently of one another, are H or alkyl radicals having 1 to 20 carbon atoms, with the proviso that R³ and R⁴ together have at least 6 and preferably 8 to 20, specifically 10 to 16, carbon atoms. As regards preparation and use, reference may be made to the review article "J. Am. Oil. Chem. Soc.", 55, 70 (1978). The olefinsulfonates can be in the form of alkali metal, alkaline earth metal, ammonium, alkylammonium, alkanolammonium or glucammonium salts. Preferably, the olefinsulfonates are in the form of sodium salts. The hydrolyzed alpha-olefinsulfonation product, i.e. the alpha-olefinsulfonates, are composed of ca. 60% by weight of alkanesulfonates and ca. 40% by weight of hydroxyalkanesulfonates; of this, 80 to 85% by weight are monosulfonates and 15 to 20% by weight are disulfonates.

Preferred methyl ester sulfonates (MES) are obtained by sulfonation of the fatty acid methyl esters of vegetable or animal fats or oils. Preference is given to methyl ester sulfonates from vegetable fats and oils, e.g. from rapeseed oil, sunflower oil, soybean oil, palm oil, coconut fat, etc.

Preferred alkylsulfates are sulfates of fatty alcohols of the general formula R⁶-O-SO₃Y, in which R⁶ is a linear or branched, saturated or unsaturated hydrocarbon radical having 6 to 22 carbon atoms and Y is an alkali metal, the monovalent charge equivalent of an alkaline earth metal, ammonium, mono-, di-, tri- or tetralkylammonium, alkanolammonium or glucammonium. Suitable fatty alcohol sulfates are preferably obtained by sulfation of native fatty alcohols or synthetic oxoalcohols and subsequent neutralization. Typical examples of fatty alcohol sulfates are the sulfation products of caproic alcohol, caprylic alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, behenyl alcohol and elaeostearyl alcohol, and the salts and mixtures thereof. Preferred salts of the fatty alcohol sulfates are the sodium and potassium salts, in particular the sodium salts. Preferred mixtures of the fatty alcohol sulfates are based on industrial alcohol mixtures which are formed e.g. during the high-pressure hydrogenation of industrial methyl esters based on fats and oils or during the hydrogenation of aldehydes from the oxo synthesis or during the dimerization of unsaturated fatty alcohols. Preference is given to using fatty alcohols and fatty alcohol mixtures having 12 to 18 carbon atoms and in particular 12 to 14 carbon atoms for the preparation of alkylsulfates. Typical examples thereof are industrial alcohol sulfates based on vegetable raw materials.

Preferred sarcosinates are sodium lauroyl sarcosinate or sodium stearoyl sarcosinate.

Preferred protein fatty acid condensates are wheat-based vegetable products.

Preferred alkylphosphates are alkyl esters of mono- and diphosphoric acid.

Suitable acylglutamates are compounds of the formula (I) 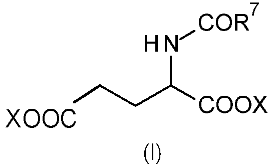 in which COR⁷ is a linear or branched acyl radical having 6 to 22 carbon atoms and 0, 1, 2 or 3 double bonds and X is hydrogen, an alkali metal, the monovalent charge equivalent of an alkaline earth metal, ammonium, alkylammonium, alkanolammonium or glucammonium. The preparation of acylglutamates takes place for example by means of the Schotten-Baumann acylation of glutamic acid with fatty acids, fatty acid esters or fatty acid halides. Acylglutamates are commercially available for example from BASF SE, Clariant AG, Frankfurt/DE, or from Ajinomoto Co. Inc., Tokyo/JP. An overview of the preparation and properties of the acylglutamates can be found by M. Takehara et al. in J. Am. Oil Chem. Soc. 49 (1972) 143. Typical acylglutamates suitable as component b) are preferably derived from fatty acids having 6 to 22 and particularly preferably 12 to 18 carbon atoms. The mono- or dialkali metal salts of the acylglutamate, in particular, are used. These include e.g. (trade name of Ajinomoto, USA in brackets): sodium cocoyl glutamate (Amisoft CS-11), disodium cocoyl glutamate (Amisoft ECS-22SB), triethanolammonium cocoyl glutamate (Amisoft CT-12), triethanolammonium lauroyl glutamate (Amisoft LT-12), sodium myristoyl glutamate (Amisoft MS-11), sodium stearoyl glutamate (Amisoft HS-11 P) and mixtures thereof.

The nonionic surfactants include, for example:
- fatty alcohol polyoxyalkylene esters, for example lauryl alcohol polyoxyethylene acetate,
- alkyl polyoxyalkylene ethers which are derived from low molecular weight C₁-C₆-alcohols or from C₇-C₃₀-fatty alcohols. Here, the ether component can be derived from ethylene oxide units, propylene oxide units, 1,2-butylene oxide units, 1,4-butylene oxide units and random copolymers and block copolymers thereof. These include specifically fatty alcohol alkoxylates and oxo alcohol alkoxylates, in particular of the type RO-(R⁸O)ᵣ(R⁹O)ₛR¹⁰ where R⁸ and R⁹ independently of one another = C₂H₄, C₃H₆, C₄H₈ and R¹⁰ = H, or C₁-C₁₂-alkyl, R = C₃-C₃₀-alkyl or C₆-C₃₀-alkenyl, r and s independently of one another are 0 to 50, where both cannot be 0, such as isotridecyl alcohol and oleyl alcohol polyoxyethylene ethers,
- alkylarylalcohol polyoxyethylene ethers, e.g. octylphenol polyoxyethylene ether,
- alkoxylated animal and/or vegetable fats and/or oils, for example corn oil ethoxylates, castor oil ethoxylates, tallow fatty ethoxylates,
- glycerol esters, such as, for example glycerol monostearate,
- alkylphenol alkoxylates, such as, for example, ethoxylated isooctyl-, octyl- or nonylphenol, tributylphenol polyoxyethylene ether,
- fatty amine alkoxylates, fatty acid amide and fatty acid diethanolamide alkoxylates, in particular ethoxylates thereof,
- sugar surfactants, sorbitol esters, such as, for example, sorbitan fatty acid esters (sorbitan monooleate, sorbitan tristearate), polyoxyethylene sorbitan fatty acid esters, alkyl polyglycosides, N-alkylgluconamides,
- alkyl methylsulfoxides,
- alkyldimethylphosphine oxides, such as, for example, tetradecyldimethylphosphine oxide.

Suitable amphoteric surfactants are e.g. alkylbetaines, alkylamidopropylbetaines, alkyl-sulfobetaines, alkyl glycinates, alkyl carboxyglycinates, alkyl amphoacetates or -propionates, alkyl amphodiacetates or -dipropionates. For example, cocodimethyl-sulfopropylbetaine, laurylbetaine, cocamidopropylbetaine, sodium cocamphopropionate or tetradecyldimethylamine oxide can be used.

The cationic surfactants include, for example, quaternized ammonium compounds, in particular alkyltrimethylammonium and dialkyldimethylammonium halides and alkylsulfates, and pyridine and imidazoline derivatives, in particular alkylpyridinium halides. For example, behenyl or cetyltrimethylammonium chloride can be used. Also of suitability are so-called ester quats which are based on quaternary triethanol-methylammonium or quaternary diethanol-dimethyl-ammonium compounds with long hydrocarbon chains in the form of fatty acid esters. These include, for example, bis(acyloxyethyl)hydroxyethylammonium methosulfate. Also of suitability is Dehyquart L 80 (INCI: Dicocoylethyl hydroxyethylmonium methosulfate (and) Propylene Glycol).

### Component d)

The liquid detergent formulations of the present invention may also comprise one or more enzymes, in particular if the liquid detergent formulation is a liquid laundry detergent formulation.

Suitable enzymes are those as typically used as industrial enzymes. These include both enzymes having optimal activity in the neutral to alkaline pH range and enzymes having optimal activity in the acidic pH range.

The enzymes are preferably selected from aminopeptidases, amylases, arabinases, carbohydrases, carboxypeptidases, catalases, cellulases, chitinases, cutinases, cyclodextrin glycosyltransferases, deoxyribonucleases, esterases, galactanases, alpha-galactosidases, beta-galactosidases, glucanases, glucoamylases, alpha-glucosidases, beta-glucosidases, haloperoxidases, hydrolase invertases, isomerases, keratinases, laccases, lipases, mannanases, mannosidases, oxidases, pectinolytic enzymes, peptidoglutaminases, peroxidases, peroxygenases, phytases, polyphenol oxidases, proteolytic enzymes, ribonucleases, transglutaminases, transferases, xylanases and mixtures thereof.

The enzymes are especially selected from hydrolases, such as proteases, esterases, glucosidases, lipases, amylases, cellulases, mannanases, other glycosyl hydrolases and mixtures of the aforementioned enzymes. All these hydrolases contribute to soil dissolution and removal from protein-, grease- or starch-containing stains. It is also possible to use oxireductases for bleaching. Of particularly good suitability are active enzymatic ingredients obtained from bacterial strains or fungi such as Bacillus subtilis, Bacillus licheniformis, Streptomyceus griseus and Humicola insolens.

Examples of suitable enzymes are given hereinafter:

### Proteases:

Suitable proteolytic enzymes (proteases) may in principle be of animal, vegetable or microbial origin. Preference is given to proteolytic enzymes of microbial origin. These also include chemically or genetically modified mutants.

Preferred proteases are serine proteases, metalloproteases or trypsin-like proteases. Preference is given to using an alkaline microbial protease. Examples of alkaline proteases are subtilisins, especially those derived from *Bacillus*, e.g. subtilisin Novo, subtilisin Carlsberg, subtilisin 309, subtilisin 147 and subtilisin 168 (described in WO 89/06279). Examples of trypsin-like proteases are trypsin (e.g. porcine or bovine) and the *Fusarium* protease described, for example, in WO 89/06270.

Preferred commercially available proteases include the proteases available under the following brand names: Alcalase^{™}, Savinase^{™}, Primase^{™}, Durazym^{™}, Esperase^{™}, Neutrase^{™} from Novozymes A/S (Denmark), the products sold by DuPont/Genencor under the Maxatase^{™}, Maxacal^{™}, Maxapem^{™}, PREFERENZ^{™} P, EXCELLENZ^{™} P Properase^{™}, Purafect^{™} and Purafect^{™} OXP brand names, and the products sold by Solvay Enzymes under the Opticlean^{™} and Optimase^{™} brand names.

### Lipases:

Suitable lipases may in principle originate from bacteria or fungi. These also include chemically or genetically modified mutants.

Examples of suitable lipases are *Humicola lanuginosa* lipase, described, for example, in EP 258 068 und EP 305 216, *Rhizomucor miehei* lipase, described, for example, in EP 238 023, *Candida* lipase, such as C. *antarctica* lipase, for example the C. *antarctica* lipases A or B as described in EP 214 761, *Pseudomonas lipase* such as P. *alcaligenes* and P. *pseudoalcaligenes* lipase, described, for example, in EP 218 272, P. *cepacia* lipase, described, for example, in EP 331 376, *P. stutzeri* lipase, described, for example, in GB 1,372,034, *P. fluorescens* lipase, *Bacillus lipase*, for example a *B. subtilis* lipase (Dartois et al., (1993), Biochemica et Biophysica acta 1131, 253-260), B*. stearothermophilus* lipase (JP 64/744992) and *B. pumilus* lipase (WO 91/16422).

In addition, a multitude of cloned lipases is suitable, comprising *Penicillium camembertii* lipase described by Yamaguchi et al., (1991), Gene 103, 61-67), *Geotricum candidum* lipase (Schimada, Y. et al., (1989), J. Biochem., 106, 383-388), and various *Rhizopus* lipases, such as *P. delemar* lipase (Hass, M. J et al., (1991), Gene 109, 117-113), *P. niveus* lipase (Kugimiya et al., (1992), Biosci. Biotech. Biochem. 56, 716-719) and *P. oryzae* lipase.

In addition, it is possible to use other types of lipolytic enzymes, such as cutinases, for example a cutinase derived from *Pseudomonas mendocina*, as described, for example, in WO 88/09367, or a cutinase derived from *Fusarium solani pisi* (as described, for example, in WO 90/09446).

Especially suitable lipases are MI Lipase^{™}, Luma Fast^{™} and Lipo-max^{™} (Genencor), Lipoclean^{™}, Lipex^{™}, Lipolex^{™} Lipolase^{™} and Lipolase Ultra^{™} (Novozymes A/S), and Lipase P "Amano" (Amano Pharmaceutical Co. Ltd.).

### Amylases:

In principle, all α- and/or β-amylases are suitable. Suitable amylases may in principle originate from bacteria or fungi. These also include chemically or genetically modified mutants.

Examples of suitable amylases are α-amylases obtained from a specific strain of B. amyloliquefaciens or B. *licheniformis*, described in detail in GB 1,296,839. Further suitable amylases comprise backbones known as SP707 from *Bacillus* sp, AP1378, BSG B. *stearothermophilus* alpha-amylase, SP690, SP722, and AA560 from *Bacillus* sp. Suitable commercially available amylases are Stainzyme^{™}, Stainzyme Plus^{™}, Natalase^{™}, Duramyl^{™}, Termamyl^{™}, Fungamyl^{™} and BAN^{™} (available from Novozymes A/S), and Rapidase^{™}, PREFERENZ^{™} S, EXCELLENZ^{™} S and Maxamyl P^{™} (available from Genencor).

### Cellulases:

In principle, all cellulases are suitable. Suitable cellulases may in principle originate from bacteria or fungi. These also include chemically or genetically modified mutants.

Suitable cellulases are described in US 4,435,307. These are fungal cellulases produced from *Humicola insolens.* Cellulases having color care properties are especially suitable. Examples of such cellulases are described in EP 0 495 257.

Suitable commercially available cellulases comprise Celluzyme^{™} produced from a strain of *Humicola insolens*, Carezyme^{™}, Celluclean^{™}, Endolase^{™}, Whitezyme^{™} (Novozymes A/S), REVITALENZ^{™} (DuPont), Biotouch C^{™} (AB Enzymes) and KAC-500(B)^{™} (Kao Corporation).

### Peroxidases/oxidases:

Suitable peroxidases/oxidases may in principle originate from plants, bacteria or fungi. These also include chemically or genetically modified mutants.

Peroxidase enzymes are used in combination with hydrogen peroxide or a hydrogen peroxide source (e.g. a percarbonate, perborate or persulfate). Oxidase enzymes are used in combination with oxygen. Both enzyme types are used for "solution bleaching", in order to avoid dye transfer from a colored fabric to another fabric when they are washed together in a liquor. They can preferably be used together with action improvers described, for example, in WO 94/12621 and WO 95/01426.

### Lyases:

In principle, all lyases are suitable. Suitable lyases may in principle originate from bacteria or fungi. These also include chemically or genetically modified mutants.

Suitable lyases are pectate lyases or pectin lyases. Suitable commercially available lyases comprise XPect^{™} (Novozymes A/S) and PREFERENZ^{™} F (DuPont).

Inventive formulation may comprise further enzymes encompassed by the term hemicellulases. These include, for example, mannanases, xanthan lyases, pectinylases (= pectinases), pectin esterases, xyloglucanases (= xylanases), pullulanases and β-glucanases.

Preferred enzymes are selected from proteases, lipases, amylases, cellulsases, lyases, peroxidases/oxidases and mixtures thereof, in particular from the group consisting proteases, amylases, cellulases, lipases, pectin lyases and mixtures thereof. If enzymes are present, the liquid detergent formulations preferably comprises at least one protease and/or amylase.

If the liquid detergent formulations contain an enzyme mixture, preference is given, for example, to enzyme mixtures of the following enzymes:
- protease and amylase,
- protease and lipase (or lipolytic enzymes),
- protease and cellulase,
- amylase, cellulase and lipase (or lipolytic enzymes),
- protease, amylase and lipase (or lipolytic enzymes),
- protease, lipase (or lipolytic enzymes) and cellulase.

The enzymes may be adsorbed on carrier substances in order to protect them from premature breakdown.

If the liquid detergent formulations contain an enzyme or enzyme mixture, the liquid detergent formulations may also comprise enzyme stabilizers. Examples of these include calcium propionate, sodium formate, boric acids, boronic acids and salts thereof, such as 4-formylphenylboronic acid, peptides and peptide derivatives, for example peptide aldehydes, polyols such as propane-1,2-diol, and mixtures thereof.

### Component e)

Builders, which are sometimes also referred to as sequestrants, builder materials, complexing agents, chelators, chelating agents or softeners, bind alkaline earth metals and other water-soluble metal salts without precipitating. They help to break up soil, disperse soil components, help to detach soil and in some cases themselves have a washing effect. In addition, when they are solid and are used in pulverulent formulations, they keep the powder free-flowing.

Suitable builders may be either organic or inorganic in nature. Examples are aluminosilicates, carbonates, phosphates and polyphosphates, polycarboxylic acids, polycarboxylates, hydroxycarboxylic acids, phosphonic acids, e.g. hydroxyalkylphosphonic acids, phosphonates, aminopolycarboxylic acids and salts thereof, and polymeric compounds containing carboxylic acid groups and salts thereof.

Suitable inorganic builders are, for example, crystalline or amorphous aluminosilicates having ion-exchanging properties, such as zeolites. Various types of zeolites are suitable, especially zeolites A, X, B, P, MAP and HS in the sodium form thereof, or in forms in which Na has been partially exchanged for other cations such as Li, K, Ca, Mg or ammonium. Suitable zeolites are described, for example, in US-A-4604224. Crystalline silicates suitable as builders are, for example, disilicates or sheet silicates, e.g. 5-Na₂Si₂O₅ or B-Na₂Si₂O₅ (SKS 6 or SKS 7). The silicates can be used in the form of their alkali metal, alkaline earth metal or ammonium salts, preferably as sodium, lithium and magnesium silicates. Amorphous silicates, for example sodium metasilicate which has a polymeric structure, or amorphous disilicate (Britesil^{®} H 20, manufacturer: Akzo), are likewise usable. Among these, preference is given to sodium disilicate.

Suitable inorganic builder substances based on carbonate are carbonates and hydrogencarbonates. These can be used in the form of their alkali metal, alkaline earth metal or ammonium salts. Preference is given to using sodium, lithium and magnesium carbonates or sodium, lithium and magnesium hydrogencarbonates, especially sodium carbonate and/or sodium hydrogencarbonate.

Customary phosphates used as inorganic builders are alkali metal orthophosphates and/or polyphosphates, for example pentasodium triphosphate.

Suitable organic builders are, for example, C₄-C₃₀-di-, -tri- and -tetracarboxylic acids, for example succinic acid, propanetricarboxylic acid, butanetetracarboxylic acid, cyclopentanetetracarboxylic acid, and alkyl- and alkenylsuccinic acids having C₂-C₂₀-alkyl or -alkenyl radicals.

Suitable organic builders are also hydroxycarboxylic acids and polyhydroxycarboxylic acids (sugar acids). These include C₄-C₂₀-hydroxycarboxylic acids, for example malic acid, tartaric acid, gluconic acid, mucic acid, lactic acid, glutaric acid, citric acid, tartronic acid, glucoheptonic acid, lactobionic acid, and sucrosemono-, -di- and tricarboxylic acid. Among these, preference is given to citric acid and salts thereof.

Suitable organic builders are also phosphonic acids, for example hydroxyalkylphosphonic acids, aminophosphonic acids and the salts thereof. These include, for example, phosphonobutanetricarboxylic acid, aminotris(methylenephosphonic acid), ethylenediaminetetraethylenephosphonic acid, hexamethylenediaminetetramethylenephosphonic acid, diethylenetriaminepentamethylenephosphonic acid, morpholinomethanediphosphonic acid, 1-hydroxy-C₁-to C₁₀-alkyl-1,1-diphosphonic acids such as 1-hydroxyethane-1,1-diphosphonic acid. Among these, preference is given to 1-hydroxyethane-1,1-diphosphonic acid and salts thereof.

Suitable organic builders are additionally aminopolycarboxylic acids, such as nitrilotriacetic acid (NTA), nitrilomonoacetic dipropionic acid, nitrilotripropionic acid, β-alaninediacetic acid (β-ADA), ethylenediaminetetraacetic acid (EDTA), diethylenetriaminepentaacetic acid, 1,3-propylenediaminetetraacetic acid, 1,2-propylenediaminetetraacetic acid, N-(alkyl)ethylenediaminetriacetic acid, N-(hydroxyalkyl)ethylenediaminetriacetic acid, ethylenediaminetriacetic acid, cyclohexylene-1,2-diaminetetraacetic acid, iminodisuccinic acid, ethylenediaminedisuccinic acid, serinediacetic acid, isoserinediacetic acid, L-asparaginediacetic acid, L-glutaminediacetic acid, methylglycinediacetic acid (MGDA), and the salts of the aforementioned aminopolycarboxylic acids. Among these, preference is given to L-glutaminediacetic acid, methylglycinediacetic acid and salts thereof.

Suitable organic builders are additionally polymeric compounds containing carboxylic acid groups, such as acrylic acid homopolymers. These preferably have a number-average molecular weight in the range from 800 to 70 000 g/mol, more preferably from 900 to 50 000 g/mol, particularly from 1000 to 20 000 g/mol, especially 1000 to 10 000 g/mol. The term "acrylic acid homopolymer" also comprises polymers in which some or all of the carboxylic acid groups are in neutralized form. These include acrylic acid homopolymers in which some or all of the carboxylic acid groups are in the form of alkali metal salts or ammonium salts. Preference is given to acrylic acid homopolymers in which the carboxylic acid groups are protonated or in which some or all of the carboxylic acid groups are in the form of sodium salts.

Suitable polymeric compounds containing carboxylic acid groups are also oligomaleic acids, as described, for example, in EP-A 451 508 and EP-A 396 303.

Suitable polymeric compounds containing carboxylic acid groups are also terpolymers of unsaturated C₄-C₈-dicarboxylic acids, where the polymerized comonomers may include monoethylenically unsaturated monomers from group (i) specified below in amounts of up to 95% by weight, from group (ii) in amounts of up to 60% by weight and from group (iii) in amounts of up to 20% by weight. Suitable unsaturated C₄-C₈-dicarboxylic acids in this context are, for example, maleic acid, fumaric acid, itaconic acid and citraconic acid. Preference is given to maleic acid. Group (i) comprises monoethylenically unsaturated C₃-C₈-monocarboxylic acids, for example acrylic acid, methacrylic acid, crotonic acid and vinylacetic acid. From group (i), preference is given to using acrylic acid and methacrylic acid. Group (ii) comprises monoethylenically unsaturated C₂-C₂₂-olefins, vinyl alkyl ethers having C₁-C₈-alkyl groups, styrene, vinyl esters of C₁-C₈-carboxylic acids, (meth)acrylamide and vinylpyrrolidone. From group (ii), preference is given to using C₂-C₆-olefins, vinyl alkyl ethers having C₁-C₄-alkyl groups, vinyl acetate and vinyl propionate. If the polymers of group (ii) comprise vinyl esters in polymerized form, they may also be present partly or fully hydrolyzed to vinyl alcohol structural units. Suitable co- and terpolymers are known, for example, from US-A 3887806 and DE-A 4313909. Group (iii) comprises (meth)acrylic esters of C₁-C₈ alcohols, (meth)acrylonitrile, (meth)acrylamides of C₁-C₈ amines, N-vinylformamide and N-vinylimidazole.

Suitable polymeric compounds containing carboxylic acid groups are also homopolymers of the monoethylenically unsaturated C₃-C₈-monocarboxylic acids, for example acrylic acid, methacrylic acid, crotonic acid and vinylacetic acid, especially of acrylic acid and methacrylic acid, copolymers of dicarboxylic acids, for example copolymers of maleic acid and acrylic acid in a weight ratio of 10:90 to 95:5, more preferably those in a weight ratio of from 30:70 to 90:10 with molar masses of from 1000 to 150 000; terpolymers of maleic acid, acrylic acid and a vinyl ester of a C₁-C₃-carboxylic acid in a weight ratio of from 10 (maleic acid):90 (acrylic acid + vinyl ester) to 95 (maleic acid):10 (acrylic acid + vinyl ester), where the weight ratio of acrylic acid to the vinyl ester may vary within the range from 30:70 to 70:30; copolymers of maleic acid with C₂-C₈-olefins in a molar ratio of from 40:60 to 80:20, particular preference being given to copolymers of maleic acid with ethylene, propylene or isobutene in a molar ratio of 50:50.

Suitable polymeric compounds containing carboxylic acid groups are also copolymers of 50% to 98% by weight of ethylenically unsaturated weak carboxylic acids with 2% to 50% by weight of ethylenically unsaturated sulfonic acids, as described, for example, in EP-A-0877002. Suitable weak ethylenically unsaturated carboxylic acids are especially C₃-C₆-monocarboxylic acids, such as acrylic acid and methacrylic acid. Suitable ethylenically unsaturated sulfonic acids are 2-acetylamidomethyl-1-propanesulfonic acid, 2-methacrylamido-2-methyl-1-propanesulfonic acid, 2-methacrylamido-2-hydroxypropanesulfonic acid, allylsulfonic acid, methallylsulfonic acid, allyloxybenzenesulfonic acid, methallyloxybenzenesulfonic acid, 2-hydroxy-3-(2-propenyloxy)propanesulfonic acid, 2-methyl-2-propene-1-sulfonic acid, styrenesulfonic acid, vinylsulfonic acid, 3-sulfopropyl acrylate, 3-sulfopropyl methacrylate, sulfomethylacrylamide, sulfomethylmethacrylamide and salts of these acids. The copolymers may also comprise, in copolymerized form, 0 to 30% by weight of ethylenically unsaturated C₄-C₈-dicarboxylic acids, such as maleic acid, and 0 to 30% by weight of at least one monomer which is copolymerizable with the aforementioned monomers. The latter are, for example, C₁-C₄-alkyl esters of (meth)acrylic acid, C₁-C₄-hydroxyalkyl esters of (meth)acrylic acid, acrylamide, alkyl-substituted acrylamide, N,N-dialkyl-substituted acrylamide, vinylphosphonic acid, vinyl acetate, allyl alcohols, sulfonated allyl alcohols, styrene and other vinylaromatics, acrylonitrile, N-vinylpyrrolidone, N-vinylformamide, N-vinylimidazole or N-vinylpyridine. The weight-average molecular weight of these copolymers is within the range from 3000 to 50 000 daltons. Particularly suitable copolymers are those with 77% by weight of at least one ethylenically unsaturated C₃-C₆-monocarboxylic acid and 23% by weight of at least one ethylenically unsaturated sulfonic acid.

Graft polymers of unsaturated carboxylic acids onto low molecular weight carbohydrates or hydrogenated carbohydrates (cf. US-A 5227446, DE-A 4415623 and DE-A 4313909) are likewise suitable. Suitable unsaturated carboxylic acids in this context are, for example, maleic acid, fumaric acid, itaconic acid, citraconic acid, acrylic acid, methacrylic acid, crotonic acid and vinylacetic acid and also mixtures of acrylic acid and maleic acid, which are grafted on in amounts of 40% to 95% by weight, based on the component to be grafted. For modification, it is additionally possible for up to 30% by weight, based on the component to be grafted, of further monoethylenically unsaturated monomers to be present in polymerized form. Suitable modifying monomers are the abovementioned monomers of groups (ii) and (iii). Suitable graft bases are degraded polysaccharides, for example acidically or enzymatically degraded starches, inulins or cellulose, protein hydrolyzates and reduced (hydrogenated or hydrogenatingly aminated) degraded polysaccharides, for example mannitol, sorbitol, aminosorbitol and N-alkylglucamine, as are polyalkylene glycols with molar masses of up to M_{w} = 5000, for example polyethylene glycols, ethylene oxide/propylene oxide or ethylene oxide/butylene oxide or ethylene oxide/propylene oxide/butylene oxide block copolymers and alkoxylated mono- or polyhydric C₁-C₂₂ alcohols (cf. US-A-5756456).

Likewise suitable are polyglyoxylic acids, as described, for example, in EP-B 001004, US- A-5399286, DE-A-4106355 and EP-A-656914. The end groups of the polyglyoxylic acids may have different structures.

Additionally suitable are polyamidocarboxylic acids and modified polyamidocarboxylic acids; these are, for example, known from EP-A-454126, EP-B-511037, WO-A-94/01486 and EP-A-581452.

Polyaspartic acids or cocondensates of aspartic acid with further amino acids, C₄-C₂₅ mono- or -dicarboxylic acids and/or C₄-C₂₅ mono- or -diamines can also be used as polymeric compounds containing carboxylic acid groups. Particular preference is given to using polyaspartic acids which have been prepared in phosphorus acids and have been modified with C₆-C₂₂ mono- or -dicarboxylic acids or with C₆-C₂₂ mono- or -diamines.

Among the polymeric compounds containing carboxylic acid groups, polyacrylic acids are preferred, including in partly or fully neutralized form.

Suitable organic builders are also iminodisuccinic acid, oxydisuccinic acid, aminopolycarboxylates, alkyl polyaminocarboxylates, aminopolyalkylenephosphonates, polyglutamates, hydrophobically modified citric acid, for example agaric acid, poly-α-hydroxyacrylic acid, N-acylethylenediamine triacetates such as lauroylethylenediamine triacetate and alkylamides of ethylenediaminetetraacetic acid, such as EDTA tallow amide.

In addition, it is also possible to use oxidized starches as organic builders.

If builders are present, is given to formulations, which contain a mixture of different builders.

The mixture of different builders preferably comprises at least two of the following constituents: at least one carbonate (e.g. sodium carbonate), at least one silicate (e.g. sodium disilicate), at least one polymeric compound containing carboxylic acid groups or at least one polymeric compound which contains carboxylic acid groups of which all or some are present in neutralized form (e.g. polyacrylic acid), at least one (poly)hydroxycarboxylic acid or a salt thereof (e.g. citric acid or a citrate), at least one aminopolycarboxylic acid or a salt thereof (e.g. methylglycinediacetic acid or a salt thereof, e.g. a sodium salt thereof), at least one phosphonic acid (e.g. 1-hydroxyethane-1-(1,1-diphosphonic acid); HEDP), at least one phosphate. More preferably, the mixture comprises at least one carbonate, at least one silicate and at least one polymeric, optionally (partially) neutralized compound containing carboxylic acid groups, and optionally at least one of the following constituents: at least one (poly)hydroxycarboxylic acid or a salt thereof, at least one phosphonic acid, at least one phosphate. The mixture especially comprises at least one carbonate, at least one silicate, at least one polymeric, optionally (partially) neutralized compound containing carboxylic acid groups, at least one (poly)hydroxycarboxylic acid or a salt thereof, and at least one phosphonic acid, and optionally at least one phosphate.

In such a mixture, the constituents are present preferably in the following amounts:

| | |
|---|---|
| e1) | at least one carbonate: 10% to 50% by weight; |
| e2) | at least one silicate: 1% to 10% by weight; |
| e3) | at least one polymeric, optionally (partially) neutralized compound containing carboxylic acid groups: 5% to 20% by weight; |
| e4) | at least one (poly)hydroxycarboxylic acid or a salt thereof: 0% to 50% by weight; |
| e5) | at least one aminopolycarboxylic acid or a salt thereof: 0% to 60% by weight; |
| d6) | at least one phosphonic acid: 0.2% to 1% by weight; |
| e7) | at least one phosphate: 0% to 60% by weight. |

The percentages by weight are each based on the total weight of the builder. The weights of e1) to e7) add up to 100% by weight.

The inventive liquid detergent formulations generally already have advantageous rheological properties because of the polymer compositions present in the form of liquid and especially aqueous composition.

In order to impart the desired viscosity to liquid of the inventive liquid detergent formulations, it is additionally possible to use at least one thickener (component g1).

In principle, any known thickeners (rheology modifiers) are suitable, provided that they do not exert any adverse effect on the action of the washing and cleaning composition. Suitable thickeners may be of natural origin or synthetic in nature.

Examples of thickeners of natural origin are xanthan, carob seed flour, guar flour, carrageenan, agar, tragacanth, gum arabic, alginates, modified starches such as hydroxyethyl starch, starch phosphate esters or starch acetates, dextrins, pectins, and cellulose derivatives such as carboxymethyl cellulose, hydroxyethyl cellulose, hydroxypropyl cellulose, hydroxypropylmethyl cellulose and methyl cellulose.

Thickeners of natural origin are also inorganic thickeners, such as polysilicic acids and clay minerals, e.g. sheet silicates, and also the silicates specified for the builders.

Examples of synthetic thickeners are polyacrylic and polymethacrylic compounds, such as (partly) crosslinked homopolymers of acrylic acid, for example with an allyl ether of sucrose or pentaerythritol or homopolymers of acrylic acid crosslinked with propylene (carbomer), for example the Carbopol^{®} products from BF Goodrich (e.g. Carbopol^{®} 676, 940, 941 and 934) or the PolygelO products from 3V Sigma (e.g. Polygel^{®} DA), copolymers of ethylenically unsaturated mono- or dicarboxylic acids, for example terpolymers of acrylic acid, methacrylic acid or maleic acid with methyl or ethyl acrylate and a (meth)acrylate which is derived from long-chain ethoxylated alcohols, for example the AcusolO products from Rohm & Haas (e.g. Acusol^{®} 820 or 1206A), copolymers of two or more monomers, which are selected from acrylic acid, methacrylic acid and their C₁-C₄-alkyl esters, for example copolymers of methacrylic acid, butyl acrylate and methyl methacrylate or of butyl acrylate and methyl methacrylate, for example the Aculyn^{®} and Acusol^{®} products from Rohm & Haas (e.g. Aculyn^{®} 22, 28 or 33 and Acusol^{®} 810, 823 and 830), or crosslinked high molecular weight acrylic acid copolymers, for example copolymers, crosslinked with an allyl ether of sucrose or pentaerythritol, of C₁₀-C₃₀-alkyl acrylates with one or more comonomers which are selected from acrylic acid, methacrylic acid and their C₁-C₄-alkyl esters (e.g. Carbopol^{®} ETD 2623, Carbopol^{®} 1382 or Carbopol^{®} AQUA 30 from Rohm & Haas).

Examples of synthetic thickeners are also reaction products of maleic acid polymers with ethoxylated long-chain alcohols, for example the Surfonic L series from Texaco Chemical Co. or Gantrez AN-119 from ISP; polyethylene glycols, polyamides, polyimines and polycarboxylic acids.

Mixtures of the abovementioned thickeners are also suitable.

Preferred thickeners are xanthans and the abovementioned polyacrylic and polymethacrylic compounds.

The inventive liquid detergent formulations may comprise at least one further additive as additive g2), including in particular one or more additives selected from the group consisting of organic solvents, foam inhibitors, bases and additives which improve the performance, stability and/or esthetic impression of the liquid detergent formulations. Suitable organic solvents g2) are selected from mono- or polyhydric alcohols, alkanolamines and glycol ethers. They are preferably selected from ethanol, n- or i-propanol, butanols, glycol, propane- or butanediol, glycerol, diglycol, propyl or butyl diglycol, hexylene glycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol monomethyl or monoethyl ether, diisopropylene glycol monomethyl oder monoethyl ether, methoxy-, ethoxy- or butoxytriglycol, i-butoxyethoxy-2-propanol, 3-methyl-3-methoxybutanol, propylene glycol t-butyl ether and mixtures of these solvents.

Useful foam inhibitors or defoamers for component g2) include, for example, soaps, paraffins or silicone oils, which may optionally be applied to support materials.

Suitable bases for component g2) are alkali metal hydroxides, alkaline earth metal hydroxides, alkali metal carbonates, alkaline earth metal carbonates, ammonium carbonate, alkali metal hydrogencarbonates, alkaline earth metal hydrogencarbonates, ammonium hydrogencarbonate and mixtures thereof. Preference is given to using sodium, lithium and magnesium carbonates or sodium, lithium and magnesium hydrogencarbonates, especially sodium carbonate and/or sodium hydrogencarbonate.

In addition, the inventive liquid detergent formulations may comprise further additives g3) which further improve the performance, stability and/or esthetic properties. In general, preferred compositions comprise, in addition to the aforementioned components, at least one further additive selected from electrolytes, pH modifiers, perfume carriers, fluorescers, hydrotropes, antiredeposition agents, optical brighteners, graying inhibitors, shrink inhibitors, crease inhibitors, dye transfer inhibitors, active antimicrobial ingredients, antioxidants, corrosion inhibitors, antistats, ironing aids, hydrophobizing and impregnating agents, swell and antislip agents and UV absorbers.

In order to improve the esthetic impression of the liquid detergent formulations, they can be colored with suitable dyes. Preferred dyes, the selection of which presents no difficulty whatsoever to the person skilled in the art, have a high storage stability and insensitivity with respect to the other ingredients of the compositions and to light, and do not have any marked substantivity toward textile fibers, in order not to stain them.

### I & I cleaners

The inventive polymer dispersions are also suitable for industrial and institutional cleaners (I & I cleaners). Industrial and institutional cleaners are typically washing compositions, all-purpose cleaners, foam cleaners, gel cleaners, CIP (cleaning in place) cleaners for professional and generally automated clearing operations, for example in industrial laundries, dairies, breweries, the food and drink industry, the pharmaceutical industry or pharmaceutical formulation, or sanitary cleaners.

The cleaners may be strongly basic with a high electrolyte content and, if required, comprise bleaches (such as hydrogen peroxide, sodium hypochlorite) or disinfectants and defoamers (for example in bottle cleaning). It is also possible for the standard aforementioned enzymes to be present in the industrial and institutional cleaners. There is a great variety in terms of the types of cleaning for which the inventive formulations are suitable. Examples include cleaning baths (stationary or mobile), spray cleaning, ultrasound cleaning, steam jet cleaning and high-pressure cleaning, optionally in combination with mechanical cleaning, for example by means of rotating brushes.

Said formulations for cleaning include those for industry, transport, commerce and industry, and for the private sector. Specific examples include: professional laundries, professional cleaning businesses, ore processing industry, metal and metalworking industry, automobile and automobile supply industry, electrical industry, electronics industry, photographic industry and businesses, leisure industry and businesses, construction material industry, brewing industry and businesses; foods industry (e.g. processing or production of meat, poultry, dairy and fish products), animal nutrition industry, cosmetics industry, pharmaceutical industry, agrochemical industry, gastronomy, the health sector, workshops, and public transport. Examples of objects to be cleaned are institutional laundry, hospital laundry, laundry from laundry collection, buildings containing living spaces, office spaces or commercial spaces of a wide variety of different kinds, and sanitary spaces, warehouses, breweries, small businesses such as bakeries, butcheries and supermarkets; hospitals, care homes, homes for the elderly, administration buildings, factory buildings, doctors' practices; and also motor vehicles (cars and trucks), buses, road tanker vehicles (interior and exterior), rail tanker wagons, passenger vehicles and goods vehicles, and aircraft and ships; and also building facades, tiled or painted walls, wooden floors (parquet, boards) with screed or textile or plastics coverings, signaling and lighting installations, furniture, railings, overhead signage, other signage, safety reflectors, delineating markers, tanks, dishware, glass panes, roads and paths, outside paving, road and railway tunnels.

### Cosmetic preparations:

The polymer dispersions described herein are also suitable as opacifiers in liquid cosmetic preparations, in particular liquid aqueous cosmetic preparations. Examples of liquid cosmetic preparations include, but are not limited to hair shampoos, hair lotions, foam baths, shower baths, oral and dental care products, creams, gels and lotions for skin care and cleansing products.

The preparations contain water and the polymer dispersion described herein and usually one ore more further ingredients conventionally present in such cosmetic preparations. Such further ingredients include but are not limited to surfactants oil, components, emulsifiers, consistency factors, thickeners, superfatting agents, stabilizers, polymers, silicone compounds, fats, waxes, pearlizing waxes, lecithins, phospholipids, biogenic agents, UV protection factors, antioxidants, deodorants, antiperspirants, antidandrufi' agents, film formers, swelling agents, insect repellents, self-tanning agents, tyrosine inhibitors (depigmenting agents), hydrotropes, solubilizers, preservatives and perfume.

The invention is described in more detail in the following examples.

### EXAMPLES

The following abbreviations are used:
- MD:: Maltodextrose
- Fe-Kat:: EDTA-Fe complex
- SDS:: Sodium dodecyl sulfate
- NaPS:: Sodium peroxodisulfate
- MAA:: Methacrylic acid
- TBHP:: tert-Butyl hydroperoxide
- H₂O₂:: Hydrogenperoxide
- AB:: Acetone bisulfit
- NaOH:: Sodium hydroxide
- % b.w.:: % by weight
- pphm:: Parts by weight per 100 parts of monomers
- rpm:: Rotation per minute
- SC:: Solids content

In the following experiments the reactants were used as such, if not stated otherwise.

Fe-Kat was used as a liquid composition having an Fe content of 6.1% by weight.

SDS was used as a 15% b.w. aqueous solution.

NaPS was used as a 2.5% b.w. aqueous solution.

TBHP was used as a 10% b.w. aqueous solution

NaOH was used as a 10% b.w. aqueous solution

AB was used as a 3% b.w. aqueous solution

H₂O₂ was used as a 35% b.w. aqueous solution

### Characterization of Maltodextrine types

Maltodextrine types have been characterized by their reductive dextrose equivalent as of solid maltodextrine (Luff-Schoorl and Lane-Eynon).

The dextrose equivalent (Lane and Eynon) methods for Corn Syrup (E-26) and Corn Sugar (F-22) are described in https://corn.org/wp-content/uploads/2018/06/Dextrose2.pdf of 4-15-2010. Methods for determination of the Dextrose Equivalent (Luff-Schoorl and Lane Eynon) are also described in No L 239/24 Official Journal of the European Communities 22.9.79. (79/78 6/EEC).

Maltodextrine types have been characterized by the viscosity of their 50 % b.w. aqueous solutions at 20°C (Brookfield Viscosity; spindle 2 at 1, 5, 20 and 100 rpm).

The properties are summarized in table 1:

**Table 1: Properties of different Maltodextrine Types (MD)**

| Maltodextrine Types | Viscosity of 50 w% solution [mPas] | | | | DE¹⁾ [meq/g] | DE²⁾ | Dextrose (DP1) [% b.w.] | Biose (DP2) [% b.w.] | Triose (DP3) [% b.w.] | Oligodextrose (DP4+) [% b.w.] | Calculated MW [g/mol] ³⁾ | SC [% b.w.] |
|---|---|---|---|---|---|---|---|---|---|---|---|---|
| | 1/5/20/100 rpm | | | | | | | | | | | |
| **A** | 1 rpm | 5 rpm | 20 rpm | 100 rpm | | | | | | | | |
| **A** | 80 | 48 | 42 | 78 | 4.4 | 28.0 | 3.0 | 11.0 | 16.5 | 69.5 | 643 | 95 |
| **B** | 200 | 96 | 86 | 122 | 3.0 | 17.3 | 1.0 | 6.5 | 11.0 | 81.5 | 1040 | 95 |
| **C** | 240 | 104 | 86 | 122 | 3.3 | 17.9 | 1.0 | 6.0 | 9.0 | 84.0 | 1006 | 95 |
| **D** | 200 | 88 | 68 | 104 | 3.6 | 21.8 | 2.0 | 7.0 | 10.0 | 81.0 | 826 | 95 |
| **E** | 160 | 56 | 44 | 76 | 6.9 | 37.7 | 1.5 | 35.0 | 21.0 | 42.5 | 477 | 95 |
| **F** | 280 | 160 | 138 | 164 | 3.8 | 22.1 | n.a. | n.a. | n.a. | 76.4 | 814 | 66.6 |
| **G** | 120 | 56 | 44 | 75 | 6.6 | 34.8 | 1.5 | 27.0 | 21.0 | 50.5 | 517 | 95 |

| | | | | | | | | | | | | |
|---|---|---|---|---|---|---|---|---|---|---|---|---|
| n.a.: not analyzed DP(1): Dextrose polymerisation degree 1) DE according to Luff-Schoorl 2) DE according to Lane and Eynon 3) Number average molecular weight, calculated from the DE according to Luff-Schoorl | | | | | | | | | | | | |

Number average molecular weight (Mn), the weight average molecular weight (Mw) of maltodextrins and also of the polymer dispersions of the present invention can also be determined using gel permeation chromatography (GPC), carried out under the following conditions:

| | |
|---|---|
| Columns: | 1 column measuring 8x50 mm, filled with a polyester copolymer (column type: GRAM precolumn), |
| | 1 column measuring 8×300 mm, filled with a polyester copolymer (column type: GRAM 30A) |
| | 2 columns measuring 8×300 mm, filled with a polyester copolymer (column type: GRAM 1000A); |
| Eluent: | dimethylacetamide + 0.5% LiBr. |
| Column temperature: | 40°C |
| Detection: | DRI Agilent 1200 |
| Flow rate: | 1 mL/min, pump (for example ERC 64.00) |
| Injection volume: | 100 µl |
| Concentration: | 4 mg/mL |
| Sample preparation: | Sample concentrations were filtered prior to analysis using Sartorius Minisart RC 25 (0.2 µm) |
| Calibration: | calibration was obtained with narrow molar mass polymethylmethacrylate standards (molar mass range 800-2200000 g/mol, PSS). Extrapolation was used to estimate the molecular weight distribution outside the range of these calibration standards with respect to the exclusion and permeation limits. |

### General procedure of the emulsion polymerization:

In a glass laboratory reactor (3000 ml) with a double jacked cooling/heating capability and continuous feed opportunities for monomers. An initial charge was given comprising the components:

### Initial Charge

▪ Water
▪ Maltodextrin
▪ Fe-Kat
▪ optionally H₂O₂

The reactor was degassed 2 times and loaded with nitrogen. The reactor was heated to 50°C at 210 rpm (U-shaped stirrer) and 2% of Feed 1 together with 10% of initiator Feed 2 was given as one shot:
**Feed 1 (aqueous emulsion)**
   ▪ Water
   ▪ Styrene
   ▪ SDS (15% b.w.)
**Feed 2**
   ▪ NaPS (2.5% b.w.)
   The reaction temperature was increased to 75°C for 15 min to finalize the prepolymerization. Than simultaneous dosage was initiated over time with rest: Feed 1 (180 min) and Feed 2 (255 min). After Feed 1 stopped: 15 min post polymerization. Than dosage of Feed 3 started (45 min):
**Feed 3**
   ▪ Styrene
   ▪ MAA
   ▪ Water
   When Feed 3 stops, dosage of Feed 4 started (one shot):
**Feed 4**
   ▪ TBHP (10 % b.w.)
   After Feed 4 stopped: 30 min post polymerization. Than dosage of Feed 5 started (90 min). After 30 min dosage of Feed 5 the Feed 6 was added (one shot).
**Feed 5**
   ▪ AB (3% b.w.)
**Feed 6**
   ▪ TBHP (10% b.w.)
   After Feed 5 stopped: 30 min post polymerization at 85°C. Cooling down to 25°C and start dosage of Feed 7 to obtain pH > 2.
**Feed 7**
   ▪ NaOH (10% b.w.)

The final dispersion was filtered over 125 micrometer filters.

The contents of the initial charge and feeds 1 to 7 of the examples 1 to 14 are summarized in the table 2. All amounts given in pphm relate relate to total amount of monomers in all feeds and are given as the solids in the respective reagent.

**Table 2: Composition of feeds and initial charge**

| Example # | 1 | 2 | 3 | 4 | 5 | 6 | 7 |
|---|---|---|---|---|---|---|---|
| Initial Charge | | | | | | | |
| Water | 100.2 | 100.2 | 66.5 | 200.4 | 200.4 | 200.4 | 200.4 |
| MD-Type /amount [pphm]¹⁾ | A/150 | A/150 | F/150 | C/150 | D/150 | B/150 | G/150 |
| Fe-Kat [pphm] | 0.002 | 0.002 | 0.002 | 0.002 | 0.002 | 0.002 | 0.002 |
| H₂O₂[pphm] | - | - | - | - | - | - | - |

| Feed 1 | | | | | | | |
|---|---|---|---|---|---|---|---|
| Water [pphm] | 95.0 | 95.0 | 95.0 | 95.0 | 95.0 | 95.0 | 95.0 |
| SDS [pphm] | 1.0 | 1.0 | 1.0 | 1.0 | 1.0 | 1.0 | 1.0 |
| Styrene [pphm] | 70.75 | 80.5 | 70.75 | 70.75 | 70.75 | 70.75 | 70.75 |

| Feed 2 | | | | | | | |
|---|---|---|---|---|---|---|---|
| NaPS [pphm] | 0.8 | 0.8 | 0.8 | 0.8 | 0.8 | 0.8 | 0.8 |

| Feed 3 | | | | | | | |
|---|---|---|---|---|---|---|---|
| Styrene [pphm] | 20.25 | 13.5 | 20.25 | 20.25 | 20.25 | 20.25 | 20.25 |
| MAA [pphm] | 9.0 | 6.0 | 9.0 | 9.0 | 9.0 | 9.0 | 9.0 |
| Water [pphm] | 3.6 | 3.6 | 3.6 | 3.6 | 3.6 | 3.6 | 3.6 |

| Feed 4 | | | | | | | |
|---|---|---|---|---|---|---|---|
| TBHP [pphm] | 0.25 | 0.25 | 0.25 | 0.25 | 0.25 | 0.25 | 0.25 |

| Feed 5 | | | | | | | |
|---|---|---|---|---|---|---|---|
| AB [pphm] | 0.45 | 0.45 | 0.45 | 0.45 | 0.45 | 0.45 | 0.45 |

| Feed 6 | | | | | | | |
|---|---|---|---|---|---|---|---|
| TBHP [pphm] | 0.15 | 0.15 | 0.15 | 0.15 | 0.15 | 0.15 | 0.15 |

| Feed 7 | | | | | | | |
|---|---|---|---|---|---|---|---|
| NaOH [pphm] | 0.2 | 0.2 | 0.2 | 0.2 | 0.2 | 0.2 | 0.2 |

| | | | | | | | |
|---|---|---|---|---|---|---|---|
| 1) Indicates the maltodextrin according to table 1 and its amount in pphm | | | | | | | |

**Continuation of table 2: Composition of feeds and initial charge**

| Example # | 8 | 9 | 10 | 11 | 12 | 13 | 14 |
|---|---|---|---|---|---|---|---|
| Initial Charge | | | | | | | |
| Water | 200.4 | 66.2 | 31.6 | 100.9 | 110.9 | 199.5 | 198.1 |
| MD-Type /amount [pphm]¹⁾ | E/150 | A/100 | A/50 | A/150 | A/150 | B/150 | B/150 |
| Fe-Kat [pphm] | 0.002 | 0.002 | 0.002 | 0.002 | 0.002 | 0.002 | 0.002 |
| H₂O₂[pphm] | - | - | - | - | | 1.0 | 2.5 |

| Feed 1 | | | | | | | |
|---|---|---|---|---|---|---|---|
| Water [pphm] | 95.0 | 95.0 | 95.0 | 95.0 | 73.6 | 95.0 | 95 |
| SDS [pphm] | 1.0 | 1.0 | 1.0 | 1.0 | 1.0 | 1.0 | 1.0 |
| Styrene [pphm] | 70.25 | 70.25 | 70.75 | 87.0 | 64.0 | 70.75 | 70.75 |

| Feed 2 | | | | | | | |
|---|---|---|---|---|---|---|---|
| NaPS [pphm] | 0.8 | 0.8 | 0.8 | 0.8 | 0.8 | 0.8 | 0.8 |

| Feed 3 | | | | | | | |
|---|---|---|---|---|---|---|---|
| Styrene [pphm] | 20.25 | 20.25 | 20.25 | 9.0 | 25.0 | 20.25 | 20.25 |
| MAA [pphm] | 9.0 | 9.0 | 9.0 | 4.0 | 11.0 | 9.0 | 9 |
| Water [pphm] | 3.6 | 2.9 | 2.9 | 2.9 | 3.6 | 3.6 | 3.6 |

| Feed 4 | | | | | | | |
|---|---|---|---|---|---|---|---|
| TBHP [pphm] | 0.25 | 0.25 | 0.25 | 0.25 | 0.25 | 0.25 | 0.25 |

| Feed 5 | | | | | | | |
|---|---|---|---|---|---|---|---|
| AB [pphm] | 0.45 | 0.45 | 0.45 | 0.45 | 0.45 | 0.45 | 0.45 |

| Feed 6 | | | | | | | |
|---|---|---|---|---|---|---|---|
| TBHP [pphm] | 0.15 | 0.15 | 0.15 | 0.15 | 0.15 | 0.15 | 0.15 |

| Feed 7 | | | | | | | |
|---|---|---|---|---|---|---|---|
| NaOH [pphm] | 0.2 | 0.2 | 0.2 | 0.2 | 0.2 | 0.2 | 0.2 |

| | | | | | | | |
|---|---|---|---|---|---|---|---|
| 1) Indicates the maltodextrin according to table 1 and its amount in pphm | | | | | | | |

### Example 15:

Example 15 was prepared according to the general procedure of examples 1 - 14 but with the exeption that feed 3 contained more water. The content of the feeds 1 to 7 and the initial charge are summarized in table 3.

### Comparative example C1:

Comparative example C1 was prepared according to the general procedure of examples 1 - 14 but without the presence of degraded starch. The content of the feeds 1 to 7 and the initial charge are summarized in table 3.

**Table 3**

| Example # | 15 | C1 |
|---|---|---|
| Initial Charge | | |
| Water | 94.3 | 32.0 |
| MD-Type /amount [pphm]¹⁾ | A/150 | - |
| Fe-Kat [pphm] | 0.002 | 0.002 |
| H₂O₂[pphm] | - | - |

| Feed 1 | | |
|---|---|---|
| Water [pphm] | 93.6 | 57.0 |
| SDS [pphm] | 1.1 | 1.0 |
| Styrene [pphm] | 70.25 | 70.25 |

| Feed 2 | | |
|---|---|---|
| NaPS [pphm] | 0.8 | 0.8 |

| Feed 3 | | |
|---|---|---|
| Styrene [pphm] | 20.25 | 20.25 |
| MAA [pphm] | 9.0 | 9.0 |
| Water [pphm] | 23.2 | 2.0 |
| SDS [pphm] | 0.4 | - |

| Feed 4 | | |
|---|---|---|
| TBHP [pphm] | 0.25 | 0.25 |

| Feed 5 | | |
|---|---|---|
| AB [pphm] | 0.45 | 0.45 |

| Feed 6 | | |
|---|---|---|
| TBHP [pphm] | 0.15 | 0.15 |

| Feed 7 | | |
|---|---|---|
| NaOH [pphm] | 0.2 | 0.2 |

### Characterization of the Polymer Dispersions

Particle size of the respective polymer dispersion was determined by static light scattering (instrument: Malvern Mastersizer 2000) of an aqueous dilution of the polymer dispersion having a concentration in the range from 0.001 to 0.1% by weight.

The opacifier effect of the respective polymer dispersion was determined by a light scattering method on an NTU scale (Nephelometer: Hanna HI88703 with accuracy ±2%; 0.02 NTU) according to the following method: 10 ml of an aqueous liquid (50% b.w. of Texapon^{®} NSO in demineralized water) is mixed with the opacifier at 0.01% solids and turbidity counts are referred to an external scale. A high value reflects a high opacifying effect.

In turbidimetry the transmission light intensity is measured with Photometer DR 6000 (Hach Lange) at 525 nm and 1 cm cuvette using an aqueous dilution of the respective polymer dispersion with solids content of 0.01 % by weight. The light diffusion factor (LD value in %) depicts how much of the light is transmitting the sample at a given cuvette length. A low value is reflecting a high opacifying effect.

Amount of coagulum was determined gravimetrically.

An overview over the polymer dispersions is given in table 4. The optical properties of the polymer dispersions are summerized in table 5.

**Table 4: overview of the examples**

| Example # | H₂O₂¹⁾ [pphm] | MD type | Amount of MD²⁾ [pphm] | MMA ³⁾ [pphm] | MD/SC ⁴⁾ [% b.w.] | SC [% b.w.] |
|---|---|---|---|---|---|---|
| C1 | 0 | -- | 0 | 9 | 0 | 39.9 |
| 1 | 0 | A | 150 | 9 | 60 | 41.8 |
| 2 | 0 | A | 150 | 6 | 60 | 41.1 |
| 3 | 0 | F | 150 | 9 | 60 | 41.6 |
| 4 | 0 | C | 150 | 9 | 60 | 40.9 |
| 5 | 0 | D | 150 | 9 | 60 | 40.6 |
| 6 | 0 | B | 150 | 9 | 60 | 40.9 |
| 7 | 0 | G | 150 | 9 | 60 | 41.2 |
| 8 | 0 | E | 150 | 9 | 60 | 41.9 |
| 9 | 0 | A | 100 | 9 | 50 | 40.4 |
| 10 | 0 | A | 50 | 9 | 33 | 40.9 |
| 11 | 0 | A | 150 | 4 | 60 | 40.6 |
| 12 | 0 | A | 150 | 11 | 60 | 41.1 |
| 13 | 1 | B | 150 | 9 | 60 | 41.7 |
| 14 | 2.5 | B | 150 | 9 | 60 | 40.8 |
| 15 | 0 | A | 150 | 9 | 60 | 42.3 |

| | | | | | | |
|---|---|---|---|---|---|---|
| 1) Amount of hydrogen peroxide in initial charge 2) Relative amount of maltodextrin to ethylenic monomers 3) Amount of methaycrylic acid in ethylenic monomers 4) Amount of maltodextrin in polymer solids | | | | | | |

**Table 5: Properties of polymer dispersion**

| | D[0.1] | D[0.5] | D[0.9] | LD¹⁾ | NTU ²⁾ | Coagulum ³⁾ |
|---|---|---|---|---|---|---|
| C1 | 152 | 230 | 347 | 43 | 183 | 20.1 |
| 1 | 81 | 190 | 1810 | 43 | 162 | 3.25 |
| 2 | 95 | 204 | 378 | 61 | 135 | 0.8 |
| 3 | 77 | 174 | 376 | 64 | 109 | 0.5 |
| 4 | 74 | 137 | 253 | 71 | 95 | 0.5 |
| 5 | 103 | 220 | 445 | 55 | 143 | 0.5 |
| 6 | 81 | 180 | 360 | 66 | 119 | 0.5 |
| 7 | 150 | 289 | 1146 | 49 | 152 | 0.5 |
| 8 | 166 | 293 | 592 | 48 | 178 | 0.5 |
| 9 | 81 | 185 | 1840 | 34 | 224 | 1.25 |
| 10 | 91 | 199 | 410 | 48 | 174 | 0.75 |
| 11 | 179 | 291 | 470 | 46 | 200 | 1.5 |
| 13 | 74 | 139 | 260 | 66 | 117 | 0.5 |
| 14 | 75 | 150 | 294 | 61 | 126 | 1.5 |
| 15 | n.d. | n.d. | n.d. | 63 | n.d. | 4.5 |

| | | | | | | |
|---|---|---|---|---|---|---|
| 1) LD value of an aqueous dilution with solids content of 0.01 % by weight 2) NTU value | | | | | | |

### Application tests:

The polymer dispersions were added to detergent formulations at room temperature with stirring at a dosage level of 1.5% active matter (solid). Stirring was continued for 30 min. Then the turbidity was measured after 24h. The following detergent formulations were used for testing:
Liquid detergent (formulation 1):
   Linear alkylbenzene sulfonate 5.5%
   soap 2.4%
   fatty alcohol ether sulfate 7.7%
   KOH 2.2%
   Nonionic surfactant 5.4%
   1,2 propylene glycol 6%
   ethanol 2%
   water ad to 100%
Hand Dish Wash detergent (formulation 2):
   fatty alcohol ether sulfate 10.5%
   betaine 11.5%
   aminoxide 7%
   ethanol 2%
   water ad to 100%
Hard Surface Cleaner (formulation 3):
   Nonionic surfactant 0.2%
   fatty alcohol ether sulfate 0.3%
   ethanol 3%
   Dipropylene glycol-n-propylether 2%
   Water ad to 100%

The NTU-values (0.1% in formulation) are summarized in table 6.

**Table 6:**

| Example # | Formulation 1 | Formulation 2 | Formulation 3 |
|---|---|---|---|
| C1 | 2094 | 2230 | 2495 |
| 1 | 2109 | 2821 | 3681 |
| 5 | 1874 | 2411 | 3218 |
| 6 | 1653 | 2153 | 2596 |

The inventive polymer dispersions show an excellent opacifying performance in liquid detergent formulations.

Testing of the opacifier effect of a polymer composition according to the invention in comparison to a polymer composition post-added with degraded starch

### Comparative polymer composition C2

150 pphm of degraded starch was added to the comparative polymer composition C1. The light diffusion factor (LD value in %) was determined as described above at 525 nm, and 1 cm cuvette with solids content of 0.01 % by weight. The results are summarized in table 7.

**Table 7:**

| Example | Total amount of degraded starch relative to the monomer M [pphm] | Added amount of degraded starch [pphm] | LD¹ | synthetic polymer content % in solid content of dispersion |
|---|---|---|---|---|
| C1 | 0 | 0 | 30 | 100 |
| C2 | 150 | 150 | 63 | 40 |
| 15 | 150 | 0 | 45 | 40 |

| | | | | |
|---|---|---|---|---|
| 1) LD value of an aqueous dilution with solids content of 0.01% by weight | | | | |

The inventive polymer dispersions show a lower LD value reflecting a better opacifying effect than a styrene/acrylate copolymer to which degraded starch was post-added.

## Claims

1. The use of an aqueous polymer dispersion, which is obtainable by radical aqueous emulsion polymerization of
i) ethylenically unsaturated monomers M comprising
a) at least 70% by weight, based on the total weight of monomers M, of at least one monomer Ma, selected from the group consisting of monovinylaromatic monomers, and optionally
b) up to 30% by weight, based on the total weight of monomers M, of one or more ethylenically unsaturated monomers Mb, which are different from the monomer Ma;
in the presence of
ii) 80 to 200% by weight, based on the total amount of monomers M, of a degraded starch;
as an opacifier in liquid formulations.

2. The use of claim 1, where the monomer Ma is styrene or comprises at least 95% by weight of styrene, based on the total amount of monomers Ma.

3. The use of any one of the preceding claims, where the monomers Mb comprise at least one monoethylenically unsaturated acidic monomer Mb1, selected from the group consisting of monoethylenically unsaturated monocarboxylic acids having 3 to 6 carbon atoms and monoethylenically unsaturated dicarboxylic acids having 4 to 8 carbon atoms.

4. The use of claim 3, where the monomer Mb is methacrylic acid or comprises at least 95% by weight of methacrylic acid, based on the total amount of monomers Mb.

5. The use of any one of claims 3 or 4, where the monomers M comprise
a) 70 to 99.9% by weight, based on the total weight of the monomers M, of at least one monomer Ma, and
b.1) 0.1 to 30% by weight, based on the total weight of the monomers M, of at least one monomer Mb1, selected from the group consisting of monoethylenically unsaturated monocarboxylic acids having 3 to 6 carbon atoms and monoethylenically unsaturated dicarboxylic acids having 4 to 6 carbon atoms and
b.2) optionally up to 29.9% by weight, based on the total weight of monomers M, of one or more further monomers ethylenically unsaturated monomers Mb2, which are different from the monomer Ma and Mb1.

6. The use of any one of the preceding claims, where the dispersed polymer particles have a D[4,3] value as determined by static light scattering of at least 250 nm.

7. The use of any one of the preceding claims, where the degraded starch has a number average molecular weight in the range from 300 to 2000 Dalton, as determined by osmometry.

8. The use of any one of the preceding claims, where the degraded starch has a dextrose equivalent according to Lane and Eynon in the range from 2 to 40%.

9. The use of any one of the preceding claims, where the degraded starch is an enzymatically degraded starch.

10. The use of any one of the preceding claims, where the liquid formulation is a liquid detergent formulation or a liquid cosmetic preparation.

## Patentansprüche

1. Verwendung einer wässrigen Polymerdispersion, die durch radikalische wässrige Emulsionspolymerisation von Folgendem erhältlich ist:
i) ethylenisch ungesättigten Monomeren M, umfassend
a) mindestens 70 Gew.-%,bezogen auf das Gesamtgewicht der Monomere M, mindestens eines Monomers Ma, das aus der Gruppe bestehend aus monovinylaromatischen Monomeren ausgewählt ist, und gegebenenfalls
b) bis zu 30 Gew.-%, bezogen auf das Gesamtgewicht der Monomere M, eines oder mehrerer ethylenisch ungesättigter Monomere Mb, die von dem Monomer Ma verschieden sind;
in Anwesenheit von
ii) 80 bis 200 Gew.-%, bezogen auf das Gesamtgewicht der Monomere M, einer abgebauten Stärke;
als Trübungsmittel in flüssigen Formulierungen.

2. Verwendung nach Anspruch 1, wobei es sich bei dem Monomer Ma um Styrol handelt oder das Monomer Ma mindestens 95 Gew.-% Styrol, bezogen auf die Gesamtmenge der Monomere Ma, umfasst.

3. Verwendung nach einem der vorhergehenden Ansprüche, wobei die Monomere Mb mindestens ein monoethylenisch ungesättigtes saures Monomer Mb1 umfassen, das aus der Gruppe bestehend aus monoethylenisch ungesättigten Monocarbonsäuren mit 3 bis 6 Kohlenstoffatomen und monoethylenisch ungesättigten Dicarbonsäuren mit 4 bis 8 Kohlenstoffatomen ausgewählt ist.

4. Verwendung nach Anspruch 3, wobei es sich bei dem Monomer Mb um Methacrylsäure handelt oder das Monomer Mb mindestens 95 Gew.-% Methacrylsäure, bezogen auf die Gesamtmenge der Monomere Mb, umfasst.

5. Verwendung nach Anspruch 3 oder 4, wobei die Monomere M Folgendes umfassen:
a) 70 bis 99,9 Gew.-%, bezogen auf das Gesamtgewicht der Monomere M, mindestens eines Monomers Ma und
b.1) 0,1 bis 30 Gew.-%, bezogen auf das Gesamtgewicht der Monomere M, mindestens eines Monomers Mb1, das aus der Gruppe bestehend aus monoethylenisch ungesättigten Monocarbonsäuren mit 3 bis 6 Kohlenstoffatomen und monoethylenisch ungesättigten Dicarbonsäuren mit 4 bis 6 Kohlenstoffatomen ausgewählt ist, und
b.2) gegebenenfalls bis zu 29,9 Gew.-%, bezogen auf das Gesamtgewicht der Monomere M, eines oder mehrerer weiterer Monomere ethylenisch ungesättigter Monomere Mb2, die von dem Monomer Ma und Mb1 verschieden sind.

6. Verwendung nach einem der vorhergehenden Ansprüche, wobei die dispergierten Polymerpartikel einen D[4,3]-Wert gemäß Bestimmung mittels statischer Lichtstreuung von mindestens 250 nm aufweisen.

7. Verwendung nach einem der vorhergehenden Ansprüche, wobei die abgebaute Stärke ein zahlenmittleres Molekulargewicht gemäß Bestimmung mittels Osmometrie im Bereich von 300 bis 2000 Dalton aufweist.

8. Verwendung nach einem der vorhergehenden Ansprüche, wobei die abgebaute Stärke ein Dextrose-Äquivalent gemäß Lane und Eynon im Bereich von 2 bis 40 % aufweist.

9. Verwendung nach einem der vorhergehenden Ansprüche, wobei es sich bei der abgebauten Stärke um eine enzymatisch abgebaute Stärke handelt.

10. Verwendung nach einem der vorhergehenden Ansprüche, wobei es sich bei der flüssigen Formulierung um eine Flüssigwaschmittelformulierung oder eine flüssige kosmetische Zubereitung handelt.

## Revendications

1. Utilisation d'une dispersion aqueuse de polymère, qui peut être obtenue par polymérisation radicalaire en émulsion aqueuse de
i) monomères éthyléniquement insaturés M comprenant
a) au moins 70 % en poids, sur la base du poids total de monomères M, d'au moins un monomère Ma, choisi dans le groupe constitué par des monomères monovinylaromatiques, et éventuellement
b) jusqu'à 30 % en poids, sur la base du poids total de monomères M, d'un ou de plusieurs monomères éthyléniquement insaturés Mb, différents du monomère Ma ; en présence de
ii) 80 à 200 % en poids, sur la base de la quantité totale de monomères M, d'un amidon dégradé ;
comme opacifiant dans des formulations liquides.

2. Utilisation selon la revendication 1, dans laquelle le monomère Ma est le styrène ou comprend au moins 95 % en poids de styrène, sur la base de la quantité totale de monomères Ma.

3. Utilisation selon l'une quelconque des revendications précédentes, dans laquelle les monomères Mb comprennent au moins un monomère acide monoéthyléniquement insaturé Mb1, choisi dans le groupe constitué par des acides monocarboxyliques monoéthyléniquement insaturés ayant 3 à 6 atomes de carbone et des acides dicarboxyliques monoéthyléniquement insaturés ayant 4 à 8 atomes de carbone.

4. Utilisation selon la revendication 3, dans laquelle le monomère Mb est l'acide méthacrylique ou comprend au moins 95 % en poids d'acide méthacrylique, sur la base de la quantité totale de monomères Mb.

5. Utilisation selon l'une quelconque des revendications 3 ou 4, dans laquelle les monomères M comprennent
a) 70 à 99,9 % en poids, sur la base du poids total de monomères M, d'au moins un monomère Ma, et
b.1) 0,1 à 30 % en poids, sur la base du poids total de monomères M, d'au moins un monomère Mb1, choisi dans le groupe constitué par des acides monocarboxyliques monoéthyléniquement insaturés ayant 3 à 6 atomes de carbone et des acides dicarboxyliques monoéthyléniquement insaturés ayant 4 à 6 atomes de carbone et
b.2) éventuellement jusqu'à 29,9 % en poids, sur la base du poids total de monomères M, d'un ou de plusieurs autres monomères éthyléniquement insaturés Mb2, qui sont différents des monomères Ma et Mb1.

6. Utilisation selon l'une quelconque des revendications précédentes, dans laquelle les particules de polymère dispersées ont une valeur D[4,3] telle que déterminée par diffusion statique de lumière d'au moins 250 nm.

7. Utilisation selon l'une quelconque des revendications précédentes, dans laquelle l'amidon dégradé a un poids moléculaire moyen en nombre dans la plage de 300 à 2 000 Dalton, tel que déterminé par osmométrie.

8. Utilisation selon l'une quelconque des revendications précédentes, dans laquelle l'amidon dégradé a un équivalent de dextrose selon Lane et Eynon dans la plage de 2 à 40 %.

9. Utilisation selon l'une quelconque des revendications précédentes, dans laquelle l'amidon dégradé est un amidon dégradé enzymatiquement.

10. Utilisation selon l'une quelconque des revendications précédentes, dans laquelle la formulation liquide est une formulation de détergent liquide ou une préparation cosmétique liquide.
